# EUROPEAN PATENT APPLICATION

(11) **EP 2 618 157 A1**
(43) Date of publication of application: **24.07.2013**
(21) Application number: 12000234.0
(22) Date of filing: 17.01.2012
(51) Int. Cl.: G01N 35/00, B01L 7/00

(54) **Laboratory apparatus for treating a sample reception section with a magnetic tool device, magnetic tool device, sample reception device for use with the magnetic tool device and method for performing a work step on at least one fluid sample using a magnetic field**

(71) Applicant: EPPENDORF AG, 22339 Hamburg (DE)
(72) Inventor: Andrulat, Harald, 22949 Ammersbek (DE); Ebers, Manfred, 25474 Bönningstedt (DE); Knofe, Helmut, 22844 Norderstedt (DE); Kroog, Jens-Peter, 22927 Grosshansdorf (DE); Lucke, Judith, 22765 Hamburg (DE)
(74) Representative: Kirchner, Christian

(57) **Abstract**

The invention is related to a laboratory apparatus and method for performing a work step on at least one fluid sample in at least one sample receptacle using a magnetic field, comprising at least one sample reception section, which has at least one sample reception zone and at least one engagement zone, which zones are arranged side by side along a horizontal plane, the sample reception zone being configured to receive a sample receptacle, at least one magnetic tool device, which comprises at least one engagement element for at least partly engaging with the at least one engagement zone, wherein the at least one sample reception section and the at least one magnetic tool device or engagement element are arranged movable in relation to each other between at least a first position and a second position for performing a relative movement along said plane.

## Description

The invention relates to a laboratory apparatus for treating a sample reception section with a magnetic tool device, a magnetic tool device, a sample reception device, in particular for use with the magnetic tool device, and method for performing a work step on at least one fluid sample using a magnetic field.

Such laboratory apparatus are used in chemical, biochemical, biological, medical or forensic laboratories, for example, to increase the efficiency of working on fluid samples by using a magnetic field. For example, automated pipetting systems increase the efficiency of transporting liquid samples by a guided movement of a pipetting tool between source positions and target positions with high precision, thus saving time and costs in comparison with manual operation. An example of such an apparatus is the automated pipetting system epMotion® offered by Eppendorf AG, Hamburg, Germany.

Magnetic fields can be utilized, for example, to apply a magnetic force to magnetic material in fluid samples, which are contained in receptacles made from a preferably nonferromagnetic material. Using this technique, target-capture methodologies have been developed in the field of biochemical and life-science. In this context for example, biologically activated magnetic particles are used as carriers for biological agent separation and purification. These methodologies are used by many diagnostic assays, which employ the detection of antigens (immunoassay). For nucleic acid-based assays (detection and/or purification) the magnetic particles have a cationic surface and association with the nucleic acid is achieved by salt-induced colloidal aggregation and electrostatic interaction.

Magnetic particles bind/associate with the respective molecules of interest in solution. The magnetic particle/ molecule of interest pair/aggregate can be captured from the solution by magnetically separating the magnetic carrier particles from the solution, either by an invasively dipping a magnet tip in the solution or by concentrating and holding them in the sample receptacle by the non-invasive application of a magnetic field from outside the receptacle, while removing the solution by aspirating the same through a pipette tip.

An example for a laboratory apparatus for performing a magnetic separation of magnetic material from liquid samples is described by US 6,605,213 B1. Here, an automated analyzer contains magnetic separation wash stations. The separation stations have a pivoting magnet moving structure, which is a lever with a relatively large pivoting radius for pivoting a magnet section such that the slot-like magnet section is lifted from a first position, where sample tubes do not engage the slot, to a second position, where the sample tubes engage the slot and apply the magnetic field to the samples. The pivoting structure requires a relatively large fraction of the volume of the separation station in the bottom area of the same, in order to provide an acceptable efficient magnetic separation. Another magnetic separator with a pivoting magnet tool, which is mounted in the bottom area of the separator and which is pivoted towards the sample vessels, is known from US 2009/0130679 A1.

Another example for a magnetic microplate separator is US 5,779,907, where a magnet microplate assembly is used to separate the magnetic particles from the sample solutions, which are placed in a microplate. The microplate has an array of equally spaced wells arranged side by side under corresponding openings of a top plate of the microplate. The magnet assembly has a support plate with an array of upright cylinder-shaped magnets on top of it. Also in this case, the magnet tool can be lifted and be moved to the bottom side of the microplate by an elevator mechanism, until the magnets are arranged within the hollow spaces between the wells for applying the magnetic field to the samples.

Another known structure for performing multiple tests on biological samples, in particular the handling of magnetic particles in biological assays, is described by WO 03/090897 A1. In case that magnetic separation of particles in a sample container is needed, a magnet bar is moved with its small front face pointing towards a container, such that magnetically responsive particles within the container are attracted to a side wall thereof, which faces the front face of the magnet bar. This configuration does not offer much flexibility for a sufficient magnetic treatment of the sample. For example, the frontal movement towards a sample container under space-limited conditions is difficult, if a sample to be tested is surrounded by an array of multiple sample containers.

It is the object of the present invention to provide an efficient laboratory apparatus, magnetic tool device and sample reception device and an efficient method for performing a work step on fluid samples in at least one sample receptacle using a magnetic field.

The object is met by the laboratory apparatus according to claim 1, the magnetic tool device according to claim 18 and the method according to claim 23. Further, a sample reception device according to claim 16 is provided, which is adapted to be used with the laboratory apparatus and the method according to the invention.

The invention offers the advantage of an efficient design of the laboratory apparatus (also referred to as "the apparatus"), in particular, with an intelligent use of the space available in the apparatus. The sample reception zone and the engagement zone are arranged side by side along a horizontal plane. The relative movement of the sample reception section and the at least one engagement element or the magnetic tool device between the first and second position is performed substantially along the same plane and, preferably, causes the at least one engagement element to at least partly or completely engage, at least in the second position, with the at least one engagement zone while moving in parallel relative to at least a part of the sample reception zone. In the second position, the sample reception section can face a side surface of the engagement element. The side surface of the engagement element can provide a larger surface than the front face, the front face being at least partially perpendicular to the relative movement towards the second position. Therefore, a high flexibility is achieved for arranging a magnet element at the engagement element, in particular at the side surface of the engagement element.

Moreover, the components of the apparatus, which are involved for performing a magnetic work step on the samples, require less space than known arrangements. Said components, namely the sample reception section and the engagement elements form a rather two-dimensional, relatively flat arrangement. For example, multiple sample receptacles (the term "sample receptacles" is used synonym for sample vessels, e.g. sample tubes) can be arranged in the sample reception section, thereby forming an array, which is aligned to a horizontal plane. The relative movement can make the engagement elements or the magnetic tool device to approach the sample reception section from a side. The magnetic tool device or the at least one engagement element can be configured sufficiently small for entering and engaging with the engagement zones, e.g. the hollow spaces between the multiple receptacles, by a movement along the same plane, in horizontal direction. As a consequence, there is more space left in the areas under said plane and above said plane, which can be used to implement other functions of the laboratory apparatus. For example it is possible to arrange a mixing or a thermostating device underneath said plane. This way, the efficiency of the laboratory apparatus can be further increased.

In the present description of the invention, preferably, the term "vertical" refers to the direction in parallel to the z-axis of a Cartesian coordinate system, which is in particular in parallel to the direction of gravity, which acts on a fluid sample, which is arranged in a sample vessel in the sample reception zone. Accordingly, the horizontal direction is perpendicular to the vertical direction and is, preferably, in parallel to the x-y-plane of a Cartesian coordinate system. The top surface of the fluid sample is arranged, in particular, horizontally. The terms "upward" or "top", respectively, refer to the positive z-axis, in particular, and in opposite direction of gravity, in particular. The direction of the negative z-axis, preferably, is the direction of gravity.

The sample reception zone and the engagement zone are arranged side by side along a horizontal plane, which implies that the top surface of a fluid sample in the sample reception zone will be parallel to said plane.

The invention offers the further advantage that a substantially horizontal relative movement of a magnetic tool device does separate the magnetic material to a wall of the sample receptacles, without moving the magnetic material out of the receptacle. The magnetic material is collected on the inner side of said wall of the receptacle, which makes it easier to remove the sample solution from the receptacle and to add, for example, a different solution. In some magnetic separation arrangements of the prior art, where the magnetic field is applied under the bottom side of the receptacles, the magnetic material collected on the receptacle's bottom is always prone to be aspirated by the pipette tips. Moreover, the lateral arrangement of the sample receptacles and the magnets have the advantage, that a more 2-dimensional distribution of the magnetic material on the inner side of said wall of the receptacle can be achieved, compared to prior art arrangements with magnets positioned under the receptacles. Moreover, less contaminating substances are enclosed by a more 2-dimensional distribution of magnetic material.

The at least one sample reception zone and the at least one engagement zone are arranged side by side along a plane. This means that said plane crosses said zones. Preferably, the at least one sample reception zone and the at least one engagement zone are aligned along the plane. In particular, multiple sample reception zones are aligned along said plane. This means that a specific point of the zones lies in said plane, in a parallel plane or is tangent to said plane or to a parallel plane. Such a point can be a center point of the zone, a topmost or lowermost point or another point.

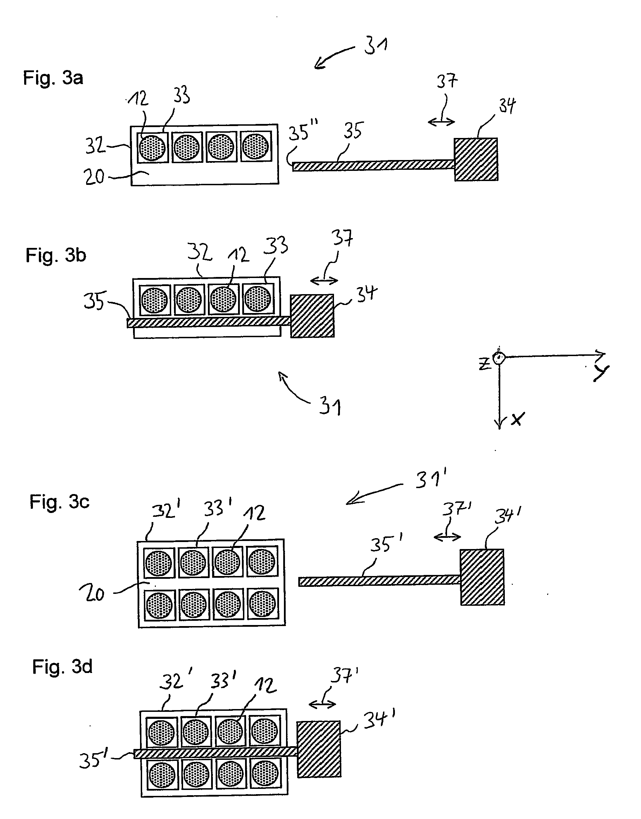

Sample receptacles, which have a longitudinal length axis through a top opening of the receptacle and which are placed in the sample reception zones, are preferably arranged having said axis substantially vertical to said horizontal plane. The plane intersects each sample reception zone, or receptacle, respectively, preferably at the same respective position. The relative movement of the sample reception section and the magnetic tool device between the first and second position is performed substantially along the plane, preferably substantially in parallel to said plane, preferably a movement along a straight line. The relative movement between the first and the second position is substantially a horizontal movement. The first and/or the second position are different positions. The relative movement can be straight (translational) but can also be bent at least in part, in particular following a pathway parallel to the plane. During the relative movement, the at least one engagement element and the at least one sample reception zone move in parallel relative to each other, which means in particular that the at least one engagement element and the at least one sample reception zone do not move on the same path, i.e. their respective paths do not cross. Further description of preferred aspects of the relative motion is given below with reference to the figures 3a to 3f.

It is possible and preferred that, in the first position, the at least one engagement element does not engage with the at least one engagement zone. Preferably, in the first position, the at least one engagement element is not engaging the at least one engagement zone and, in the second position, the at least one engagement element is at least partly or fully engaging the at least one engagement zone, which means, in particular, that in the second position, at least a part of the engagement zone or the complete engagement zone is occupied by the engagement element.

However, it is possible that, in the first position, the at least one engagement element does engage at least in part with the at least one engagement zone or does engage with a part of the at least one engagement zone. In this case, only a small relative movement is required for approaching the second position, starting from the first position.

The fluid samples, which are the targets for performing a magnetic work step (using a magnetic field), can be any liquid samples, for example, such samples, which are investigated and processed in (bio-)chemistry and life-science laboratories, for example biochemical samples containing, e.g., living cells, PCR-reagents, nucleic acids, antigens etc. In particular, the samples can contain magnetic material, for example, magnetic particles with average diameters in the order of micrometers or nanometers. Magnetic material is defined here to be a material, which can be moved by a homogeneous or inhomogeneous magnetic field. The magnetic field can be static or time variable and can be, in particular, the magnetic field of commercial permanent magnets. Preferably, the magnetic material is a ferromagnetic, paramagnetic or diamagnetic material. Magnetic material in the sample can be used, for example, to perform target-capture methodologies with the laboratory apparatus. The work step, preferably, provides the application of a magnetic field to the fluid samples. Any magnet element small enough for being arranged and applied within the engagement zone(s) adjacent to the sample reception zone(s) is appropriate to be used for the magnetic tool device. The magnetic interaction can also be electromagnetic.

The invention also refers to a magnetic tool device, in particular magnetic tool device of the laboratory apparatus according to the invention, comprising at least one engagement element, which is configured for performing a movement relative to a sample reception section, in particular the sample reception section of the laboratory apparatus according to the invention, which sample reception section provides at least one sample reception zone and at least one engagement zone, whereby these zones are arranged side by side along a horizontal plane, and the sample reception zone is configured to receive a sample receptacle, such that during the relative movement the at least one engagement element and the at least one sample reception zone move at least partly in parallel relative to each other, and wherein the at least one engagement element is configured for at least partly engaging with the at least one engagement zone, wherein, in the first position, the at least one engagement element is not arranged relative to the at least one engagement zone for performing a work step using a magnetic field, and in the second position, the at least one engagement element is at least partly engaging with the at least one engagement zone and is arranged for performing a work step using a magnetic field in the second position.

In a first preferred embodiment of the laboratory apparatus according to the invention the laboratory apparatus further comprises a base, wherein the at least one sample reception device and, respectively, the at least one sample reception section is stationary with respect to the base, at least during the relative movement or permanently, while the at least one magnetic tool device or the at least one engagement element is movable with respect to the base for performing the relative movement. Such a configuration offers the advantage, for example, that the receptacles, which are located stationary in the sample reception section, can be easily treated from the top, e.g. by a fluid transfer device, e.g. a pipetting tool, while at the same time changing the position of the magnetic tool device or engagement element, thereby controlling the performance of the non-invasive treatment during the additional work step of pipetting. Similarly, another work step, e.g. temperature control and/or mixing, could be performed more easily (because being stationary) from the bottom side of the sample reception section, while at the same time changing the position of the magnetic tool device or engagement element. All the work steps can be performed at a single position of the sample receptacles and can be performed simultaneously, at least in part or completely. Thus the sample reception section with the receptacles does not need to be moved to another position in the laboratory apparatus to perform magnetic separation work step, a thermostating work step, especially a heating work step or a mixing work step. In particular, it is possible and advantageous to perform the various combinations work step simultaneously: e.g. a magnetic work step together with thermostating, especially a heating work step in order to increase the speed of the association of the magnetic particles with its target molecules. In the first preferred embodiment, the apparatus has at least one device for transporting the magnetic tool device or at least one engagement element, wherein said transporting device can comprise a motor and/or a gear drive, and/or has at least one device for guiding the movement of the magnetic tool device, wherein said guiding device can comprise at least one rail element and/or guide bar. The first preferred embodiment offers the further advantage that the risk is reduced to damage the samples by reducing the time of transporting the samples. This way, cross contamination between multiple samples, for example, can be reduced.

The base of the apparatus can be a platform and/or a frame, which can be placed stationary with respect to a laboratory environment.

In a second preferred embodiment of the laboratory apparatus according to the invention the laboratory apparatus further comprises a base, wherein the at least one sample reception device and, respectively, the at least one sample reception section is movable with respect to the base for performing the relative movement, while the at least one magnetic tool device or the at least one engagement element is stationary with respect to the base, at least during the relative movement or permanently. In the second preferred embodiment, the apparatus has at least one device for transporting the sample receptacles and thereby, preferably, also the sample reception section, and/or a device for guiding the movement of the sample receptacles and thereby, preferably, also the sample reception section. The advantage of the second embodiment is that no additional means for transporting the magnetic tool device or transporting the engagement element is required, because said parts are stationary during the relative movement, at least in horizontal direction. Thus, the relative movement is achieved by transporting the sample reception section, or respectively, the sample receptacle device, or respectively, the sample receptacles seated in the sample reception section. However it is possible to also provide a device for transporting the magnetic tool device or engagement element. The configuration according to the second embodiment can be advantageous, moreover, in a case where the magnetic tool device is one station in a pathway of an automated multistep process for treatment of samples, where the magnetic treatment station is one of multiple stations, where a work step on the samples is performed. This way, it can be easier to allow each sample the same processing time for the magnetic work step.

In a third preferred embodiment of the laboratory apparatus according to the invention the laboratory apparatus further comprises a base, wherein the at least one sample reception device and, respectively, the at least one sample reception section and the at least one magnetic tool device or at least one engagement element are movable with respect to the base, while performing the relative movement. Such a configuration may offer the advantage of a more time-efficient multi-step processing of samples, because the transport time of sample receptacles between two different stations can be used to apply simultaneously the magnetic work-step. In the third embodiment, the apparatus has preferably, first a device for transporting the sample receptacles and, second a device for transporting the magnetic tool device, or a corresponding guiding device for guiding the respective movement.

The sample reception device can be a part, which is fastened or integral with the laboratory apparatus, or can be a separate part, which is separatable from the laboratory apparatus and which is arrangable at the laboratory apparatus, in particular in a target position or mounting position, for providing the sample reception section.

Preferably, the apparatus has a guiding device, which is configured to guide the relative movement of the least one sample reception section and the at least one magnetic tool device between the first and second position.

The laboratory apparatus can comprise a device for receiving the sample reception device, which sample reception device can comprise or consist of an individual sample tube or a multi-receptacle plate, for example. Moreover, the laboratory apparatus can comprise a device for holding the sample receptacles or for holding the sample reception device.

A sample reception zone is configured to receive a sample receptacle. This means, the sample reception zone can be a hollow space or comprise a hollow space, which is configured to receive a sample receptacle.

An engagement zone is configured to engage with at least one engagement element, which means that the engagement zone can be a hollow space or comprise a hollow space, which is configured to receive an engagement element. An engagement zone can be limited by at least one wall section, comprising, respectively preferably, a bottom wall section, a top wall section opposing the bottom wall section, a first side wall section, a second side wall section opposing the first side wall section. Said wall sections can be, respectively, a part of the sample reception device. Preferably, the engagement zone comprises at least one opening on a lateral side, for being accessible from one side by an engagement element, preferably during said horizontal relative movement. Said at least one opening can be part of the sample reception device. In particular, the wall sections can form a slot-shaped or tube-shaped engagement zone. Preferably, the bottom wall section is configured to be temperature controlled, wherein the bottom wall section comprises a material of sufficient heat conductivity, e.g. metal, e.g. aluminum, steel, silver. Preferable the bottom wall section is part of a support plate for supporting the at least one sample receptacle, part of a holding device, or part of a heat transfer device. Generally, said wall sections, respectively, can be part of a holding device, or part of a heat transfer device or part of the sample reception device.

Preferably, the sample reception section has an upper area, preferably has an intermediate area and has a bottom area, which areas are preferably provided on top of each other in vertical direction. The magnetic tool device is preferably arranged or arrangeable such that during the relative movement, the at least one engagement element engages with only one of said areas, preferably the intermediate area, but does preferably not engage with the other areas, and/or does preferably not engage with the upper area and/or does preferably not engage with the bottom area. In case that the at least one engagement element, in the second position, is not arranged in the bottom area, and is arranged preferably only in the intermediate area, the advantage is that magnetic material can be attracted away from the bottom area, by the magnetic force, such that a step of pipetting the fluid sample can be performed with a reduced risk to aspirate the magnetic material. Said upper, intermediate or bottom area of the sample reception section is preferably integral to said sample reception section and preferably has no structural delimitation within the sample reception section. However, it is possible that an area is structurally delimited within the sample reception section. For example the bottom area can be a platen, on top of which the sample reception zones are provided. Generally, the upper area can comprise a plane or a platen with at least one opening for receiving the sample reception device or the at least one sample receptacle. The intermediate area can be a cuboid-shaped horizontal central layer of the sample reception section, wherein height H**_**l of the layer (L) is smaller than the height H_s of the sample reception section (s) by a factor c, wherein H_l = c*H_s, with the following preferred lower and upper limits for the factor c: {0.05; 0.1; 0.2; 0.3; 0.4} <= c <= {0.4; 0.5; 0.6; 0.7; 0.8; 0.9; 0.95}. The height of the upper area and the lower area can be the same or different.

Using at least two of said areas, the respective area, which is not needed for receiving the engagement element(s), can be used for providing other sub-devices of the laboratory apparatus. Sub-devices for performing an invasive work step on the fluid samples in the receptacles usually require accessing the receptacles from the top. A sub-device of the apparatus can be a fluid transfer device, e.g. a pipetting device, which has to be arranged at least in part and at least temporarily in the upper area. Preferably, the laboratory apparatus comprises a fluid transfer device, which, in the second position, is arrangeable at least in part in the upper area. The fluid transfer device can comprise one or more pipette tips, needles or other tubular tools. In particular the pipette tips on the fluid transfer device are lowered at least in part into the upper area of the sample reception section, such that the pipette tip is lowered into an actual receptacle.

Analogous, the apparatus can comprise at least one further sub-device to be arranged, preferably, on the base of the apparatus, preferably underneath the bottom area or at least in part in the bottom area of the sample reception section. The sub-device can be a heat exchange device, which can be arranged contacting the bottom area or arranged at least in part in the bottom area. The heat exchange device can be or comprise or be a part of, for example, a thermorack or a heat transfer section, e.g. a heat conducting plate, or can be integrated into these parts. A thermorack is a sample holder, which has the capability for conducing heat toward -or away from- the at least one sample Preferably, the apparatus comprises a base and the heat exchange device arranged on the base and comprises a heat transfer section, which, in particular, can be part of the laboratory apparatus. The heat transfer section can comprise a heat conducting plate or structure, which comprises a material with good heat conductivity, or can substantially consist of such a material. The material can be metal, e.g. aluminum, steel or silver. Further devices for improving thermal contact between the sample receptacles and the heat exchange device can be provided, e.g. a pressure device for enhancing the thermal contact by pressing the sample reception section against the heat transfer section, or heatconductive-paste or -foil.

Preferably, the at least one engagement element of the magnetic tool device, in the second position, is positioned substantially in one of said areas and not in the other area(s) of the sample reception section. The other area(s) is (are) available for performing other work steps on the samples. In a preferred embodiment of the apparatus, the at least one engagement element of the magnetic tool device, in the second position, is positioned substantially the intermediate area and substantially not positioned in the upper area and substantially not positioned in the bottom area of the sample reception section. This way, at least three work steps can be performed simultaneously on the at least one fluid sample, for example, a fluid transfer process (e.g. in the upper area), a temperature control process (e.g. thermostating or heating in the bottom area), and the magnetic work step in the intermediate area. In an alternative routine, the bottom area is used for a mixing step, while upper area is used for fluid transfer and the intermediate area is used for the magnetic work step. Thus, a highly efficient workflow can be achieved.

Preferably the laboratory apparatus comprises a holding device for holding at least one sample receptacle in the sample reception section. The holding device can be or can be a part of the sample reception device. In a preferred embodiment, the holding device is configured to comprise or be a part of a thermorack and/or a contact area, which is arranged in the sample reception section or which provides and forms the sample reception section. Preferably, the thermorack comprises, or substantially consists of, a material, which in particular has a sufficient heat conductivity, for example metal, e.g. aluminum or silver. Preferably, the thermorack has a contact area for establishing thermal contact with a heat transfer section of the apparatus, which can be connected to a heat source and/or a heat sink. The contact area can be part of a platen, e.g. of metal, which can be integral to the thermorack or connected to the same. Thermal contact can be achieved by placing the thermorack on top of a heat exchange station, which can be part of the apparatus. The heat exchange station can comprise a temperature controlled base platen, which can be part of the heat transfer section. Temperature control can be achieved by providing at least one temperature device (=thermostating device), e.g. a peltier device, for heating and/or cooling the base platen, configured for pumping heat, e.g. for heating and/or cooling the base platen. At least one temperature sensor and electronic control loop(s) can be provided to control the temperature of the base platen and/or the temperature provided by the heat exchange station to the thermorack.

It is further possible that the thermorack comprises at least one temperature device, e.g. a peltier device, for heating and/or cooling the thermorack, thereby controlling the temperature of the thermorack, the sample receptacles and the fluid samples. Preferably, the thermorack comprises at least one temperature sensor for measuring the temperature of the thermorack, which can be used to control the thermorack temperature according to a predetermined set temperature. Preferably, the laboratory apparatus comprises electric control loops for controlling the temperature of the thermorack according to a predetermined set temperature, which can be user-defined or defined by a computer program code, for example. A thermorack, preferably, offers the advantage that the fluid samples can be kept in a temperature controlled environment, while a magnetic work step can be performed on the samples at the same time.

The thermorack, preferably, has at least two block sections or, preferably, multiple block sections, which can be part of an integral block or which are, preferably, arranged in an array, preferably a periodic array. A block section, preferably, is made from metal, in particular, from aluminum and/or silver and/or any other material with, preferably, a good thermal conductivity. The block sections can be connected to (or be integral with) a plate, which preferably has a good thermal conductivity, e.g. made from the same metal. The at least two block sections can be equally spaced apart from each other, thereby said hollow space defining the at least one engagement zone, which is required to host the engagement element of the magnetic tool device in the second position. Moreover, an engagement zone can be provided between the block sections in any way as required. The engagement zone can be a hollow space in the sample reception section or, respectively, the block. The block or the block sections, preferably, are arranged in the sample reception zone or form and/or provide the sample reception zone.

The holding device for holding the at least one sample receptacle can be, further, a plate element with openings or recesses for receiving sample tubes, which can be individually, group-wise or as a unit be suspended or supported at the plate element. The holding device can comprise at least one socket, frame, or suspension, etc. The holding device can be connected or be connectable to a device for transporting the sample reception section or the at least one sample receptacle. The device for transporting can be part of the apparatus and can comprise, for example, a movable gripper member, whose actions can be computer controlled and which can be part of a robotic movement system of the laboratory apparatus, laboratory workstation or an automated laboratory system, which comprise the laboratory apparatus.

The sample receptacles can be provided individually or as a group or a plurality of sample receptacles, which can be connected in series or in a network, e.g. in an array, forming a multi-receptacle device. Thus, a sample reception device can be configured to be a single receptacle device or a multi-receptacle device. The sample receptacles can be configured to contain typical volumes of laboratory samples, e.g. in the order of several 100 or 1000 microliters. The receptacles can be PCR tubes, standard test tubes, capped tubes. Moreover, the multi-receptacle devices can be six-well plates, 24/96/384-well plates, PCR plates, or deep-well plates, etc. Each receptacle is arranged in a sample reception zone or forms a sample reception zone, preferably, such that engagement zones are provided adjacent to the sample reception zones.

The invention is further related to a sample reception device, in particular for use with the apparatus and/or the method according to the invention.

The sample reception device can be a part, which is fastened or integral with the laboratory apparatus, or can be a separate part, which is separatable from the laboratory apparatus and which is arrangable at the laboratory apparatus, in particular in a target position or mounting position, for providing the sample reception section. The sample reception device provides the at least one sample reception section, which comprises the at least one sample reception zone and the at least one engagement zone. The sample reception device comprises at least a support for supporting at least one sample receptacle in a sample reception zone or comprises at least the holding device or preferably is the holding device, in particular a thermorack.

The sample reception device preferably is arrangeable at the laboratory apparatus according to the invention in a target position and capable of being removed from the same and which is adapted to provide, if arranged in the target position, the sample reception section of the laboratory apparatus, such that the sample reception device provides at least one sample reception zone and at least one engagement zone adapted for engagement with at least one engagement element of the laboratory apparatus, whereby these zones, namely the at least one sample reception zone and the at least one engagement zone, are arranged side by side along a horizontal plane, and the sample reception zone is configured to receive a sample receptacle.

Preferably, the sample reception device is shaped such that the at least one engagement element fits into the sample reception device. Preferably, the sample reception device is shaped such that a recess of the sample reception device or a hollow space is provided, acting as the engagement zone for receiving an engagement element. Preferably, the engagement zone is provided between at least two sample reception zones of the sample reception device, wherein the engagement zone is adapted to receive the engagement element in the second position. The sample reception device and/or the engagement element(s) can be shaped such that the engagement element or at least one magnet element, or at least two opposing magnet elements are arranged in a minimal distance d from the at least one sample reception zone, in the second position.

The magnetic force between the magnetic material in a fluid sample and the magnet element is indirect proportional to the square of the distance. Therefore, it is preferred to configure the sample reception section, the sample reception device and the engagement elements, as well as the magnetic tool device and the apparatus, respectively, such that said distance is below an upper limit. The distance d can be 0, for example, such that the engagement element contacts the sample reception device or at least one or two receptacles of the sample reception device, or the sample reception section or the sample reception zone, in particular. Preferably, the distance d is taken from the following preferred ranges, which are any combinations of the following preferred lower and upper limits: {0; 0.1; 0.2; 0.5; 1.0; 1,5; 2.0; 3.0; 4,0; 5,0 mm} <= d <= {5.0; 7.5; 10; 15; 20; 40; 50 mm}. Preferably, the width W of the at least one recess or opening in the sample reception device, preferably is chosen from the preferred ranges, which are any combinations of the following preferred lower and upper limits: {0; 0.1; 0.2; 0.5; 1.0; 1,5; 2.0; 3.0; 4,0; 5,0 mm} <= d <= {5.0; 7.5; 10; 15; 20; 40; 50 mm}, W being measured in the horizontal plane, perpendicular to the direction of the relative movement of the magnetic tool device and the sample reception section. The sample reception device can be disposable, e.g. made from plastics, or can be non-disposable, e.g. comprising metal. The sample reception device, preferably, comprises or consists of a polymer, which preferably is appropriate for fabricating the device by injection molding. Preferably, the sample reception device can forms (or is) the thermorack.

Preferably, the sample reception section has Nₐ sample reception zones, which are arranged in N_{b} parallel rows, and wherein the magnetic tool device has N_{c} engagement elements, which are configured to engage with the at least N_{d} engagement zones adjacent to the Nₐ rows in the second position, whereby N_{b}, N_{c} and N_{d} are all a natural number larger or equal than 1 and Nₐ is a natural number larger or equal than 2. Such a configuration is in particular efficient when Nₐ > N_{c}.

Preferably, a number of N1 sample reception zones and a number of N2 engagement zones are provided for the sample reception section, which zones are arranged side by side along a horizontal plane, a sample reception zone being configured to receive a sample receptacle, an engagement zone (or multiple engagement zones) being configured to receive one engagement element, N1, N2 being, respectively, natural numbers larger or equal than 2, 3, 4, 5, 6, 8, 10, 12, 16, 20 or 24, respectively, wherein preferably N1 and/or N2 is smaller or equal than 10, 20, 50, 100 or 500. This way, a magnetic field can be provided simultaneously in one work step to multiple samples, in particular, which provides high efficiency to the apparatus.

Preferably, at least two sample reception zones and at least one engagement zones are provided, wherein one engagement zone is arranged between two sample reception zones. This offers the advantage that each of two opposing side surfaces of the engagement element can be used to provide a magnetic field to a sample; in particular, a number of N3 engagement zones can be used (and the same number of N3 engagement elements can be sufficient) for applying a magnetic field to a number of at least N3 * 2 sample reception zones, N3 being a natural number and N3>= 1. Thereby, the effort for implementing the magnetic tool device is reduced.

The sample reception section can have a plurality of sample reception zones, which are arranged in a number of N4 parallel rows, preferably straight rows. Preferably, the magnetic tool device has a number of N4 * 0.5 engagement elements, which are configured to engage the at least N4 * 0.5 engagement zones between the rows, in the second position, N4 being an even natural number larger or equal than 2. Preferably, each engagement element is configured to perform the magnetic work step on the samples in the receptacles of two rows of sample reception zones. Thereby, the effort for implementing the magnetic tool device is reduced. Such a configuration offers the advantage that substantially each receptacle is facing one engagement element or at least one magnet element, respectively. The number N4 preferably is 6, which is appropriate for a sample reception section with 24 sample reception zones, which are arranged in 6 rows of 4 sample reception zones. Moreover, the number N4 preferably is between 2 and 24, preferably 2, 4, 6, 8, 10, 12. Preferably, each engagement zone of the sample reception section is shaped to be engageable by an engagement element.

Preferably, the magnetic tool device has a number of N5 or N5-1 engagement elements, which are configured to engage, in the second position, N5-1 engagement zones between the N5 rows of sample reception zones of the sample reception section. N5 can be a natural number larger or equal than 2. Such a configuration offers the advantage that substantially all receptacles of N5 or N5-1 rows of sample reception zones can be arranged such that each receptacle is facing at least two different engagement elements or magnet elements, respectively. This can be used to adapt the sequence of magnet elements in the desired manner, for example to provide permanent magnets on opposing sides of a sample vessel, which can be polarized differently.

The magnetic tool device and/or the sample reception device (or holding device for the receptacles) can be mounted, respectively, on a movable carrier member or on a stationary carrier member, or can be directly mounted to a guiding device or transport device. The apparatus can have at least one guiding device for guiding the relative movement, which can be the guiding device for guiding the motion of the magnetic tool device or the sample reception section along the plane, for example. Preferably, the guiding device has at least one guiding rail or guiding rod, wherein the tool section or sample reception section or the movable carrier member has corresponding bearing means or wheels for providing the relative movability.

Preferably, the transport device comprises a motor device or drive device for moving the magnetic tool section or sample reception section or the movable carrier member, respectively. Preferably, the operation of the motor device or drive device is controlled by a programmable computer device, which can be part of the electronic control device of the apparatus. Preferably, a coupling member is provided to mediate the movement, which is provided by the motor device or drive device, to the driven component(s), which is the magnetic tool device and/or the sample reception section and/or the movable carrier member. Preferably, the coupling member is a synchronous belt, which allows for a slipfree coupling of the movement of cam gears, which are respectively connected to the drive gear of a driving axle and the driven gear, which is connected to the driven member.

Preferably, the magnetic tool device has an arm section with one or more arm elements, wherein each arm element forms at least one engagement element. Preferably, the arm section has at least two arm elements, most preferably the arm section has three arm elements, and further preferably multiple arm elements. Each arm can be formed as a rigid rod-like element, which is configured to engage the hollow space(s) between the receptacles, preferably for being arranged (only or at least) in the intermediate area of the sample reception section. Preferably, the arm section comprises a base element and the at least one arm element is mounted laterally to the base element, the base element being preferably the carrier of the at least one arm element, preferably the at least one arm element protruding horizontally from a side of the base element. Preferably, multiple arm elements are mounted in parallel to the base element. The base element can be connected or can be integrally connected to the magnetic tool device, in particular to a carrier member of the same. Preferably, the at least one arm element is arranged horizontally and self-supporting at the base element to provide an empty space, which is located below the at least one arm element, such that this empty space can be occupied by the bottom area of the sample reception section. This configuration allows to provide additional devices for treating (e.g. for heating, mixing) samples, at the same time when performing a magnetic work step.

An engagement element or arm element can be mounted movable with respect to its carrier, namely the magnetic tool device or the arm section, along at least one direction of movement, which can be a translation or a rotation, preferably a translation along the vertical direction or the horizontal direction, which is perpendicular to said relative movement.

The magnetic tool device has at least on magnet element, preferably at least two magnet elements, and preferably a plurality of magnet elements, which are configured to provide the magnetic interaction with the at least one fluid sample.

A magnet element can comprise a permanent magnet, e.g. a ferromagnet, e.g. comprising samarium-cobalt, neodymium-iron-boron (NIB) and/or aluminum-Nickel-Cobalt. The permanent magnet can be disk shaped for mounting the permanent magnet on a side of the engagement element. The engagement element can be formed at least in part or completely from a permanent magnet and/or from a ferromagnetic material, which can be provided with a protection layer, made e.g. from plastics. The permanent magnet can be ring shaped for mounting the permanent magnet around the engagement element. The permanent magnet can be rod-shaped for mounting the permanent magnet through the engagement element. The permanent magnet can be mounted movable with respect to the apparatus, in particular in vertical direction. A magnet element can also comprise an electromagnet, e.g. a coiled electric wire with a core of ferromagnetic material, such as iron.

Preferably, the magnetic tool device has at least one magnet element and is configured such that, in the second position, the magnet element is arranged in the hollow space to apply a magnetic force to a magnetic material in the at least two fluid samples.

The number N6 of magnet elements of the magnetic tool device is preferably equal to the number N7 of sample reception zones, but can also be different, for example less than N7, in particular in the case where one magnet element is used to provide a non-invasive interaction with at least two samples (or sample receptacles or sample reception zones), wherein N6, N7 are natural numbers larger or equal than 1. Preferably, the magnet elements are arranged such at the engagement elements that the direction of the polarity of all magnet elements is the same. However, it is also possible to arrange the magnet elements in another manner, e.g. with alternating polarity on one side surface of an engagement element.

Preferably, the apparatus comprises an electric control device for controlling the functions of the apparatus and its sub-devices, for example, the activity of fluid transfer device, e.g. a pipetting device, of transport devices, motor and drive devices, of temperature controlling devices, gripper activities, etc. Preferably, the electronic devices comprises programmable circuits, calculating means, e.g. a CPU or a microprocessor, data memory means, a program code memory means, user interface means, external devices interface means, etc. Moreover, the electric control device can be configured to control the activity of the engagement elements and magnet elements, e.g. controlling the engagement start- and end time, the start- and end time of the magnetic work step, e.g. by switching on/off the electromagnetic magnet element, or by arranging a movable magnet element relative to the engagement element. Preferably, the electronic devices comprises a program code according to the invention which is adapted to let the at least one magnetic tool device or engagement element and the at least one sample reception zone of the apparatus perform a relative movement, in particular according a predetermined time schedule or sequence, which can be influenced or determined by the user.

An engagement element preferably comprises at least two magnet elements, which are mounted on opposing sides, preferably horizontally opposing side surfaces, of the engagement element, for establishing a magnetic interaction with the fluid sample in the receptacle, which faces the respective magnet element in the second position.

Preferably, the magnetic tool device is configured such that the engagement element or a part of the engagement element can be moved also along a direction perpendicular to said plane. Further, the engagement element can provide at least two or more magnet elements, which are arranged vertically on top of each other or, respectively, facing a single sample receptacle. This way, the position of the application or a variation thereof can be controlled with a spatial resolution, e.g. to collect and drag magnetic material by a vertical motion or vertically varied activity of a magnetic magnet element.

Preferably the apparatus according to the invention comprises two, three or four, or even more magnetic tool devices. With respect to a substantially cuboid-shaped sample reception section, each face side can be used to let a magnetic tool device or its at least one engagement element engage the hollow spaces between sample reception zones. This allows to perform multiple (preferably non-invasive) magnetic work steps on the sample reception section, which can be stationary, in particular.

Preferably the apparatus according to the invention comprises two, three or four, or even more sample reception sections, which can be movable, in particular. Such a configuration allows the efficient use of multiple stations for performing a work step, which can be provided at the apparatus.

Preferably, the laboratory apparatus is a laboratory workstation (hereinafter also referred to as "workstation") or is part of an automated workstation in a laboratory. An automated workstation comprises more than one internal stations, each station being related to a specific function of the apparatus. Such a station can be a storage position for storing movable components, e.g. sample receptacles, receptacle stands, liquid storage containers, waste containers, pipette tips, tools for being transported by means for transporting, e.g. a pipetting tool. Such a station can further be a station for treating samples, e.g. for performing at least one or multiple work-step(s) on the samples. Such a work-step can be a magnetic treatment of the samples, e.g. for separating magnetic particles from the samples. The work-step can be, the transfer of fluid samples, either a transfer away from the sample receptacles or a transfer into the sample receptacles, using a fluid transfer device, e.g. a pipetting tool. Another work-step is the temperature control of the samples. A further work-step is the mixing of the samples by providing a controlled oscillatory motion to the sample receptacles. For this purpose, a mixing device can be provided, which has a support member, e.g. a platen and/or a holding device, for supporting a sample receptacle or sample reception device, and a motor for performing an oscillatory motion of the support member. Moreover, the work-step can be to apply a vacuum to the samples for the purpose of filtering the samples, for example. Other work-steps within the same workstation are possible. It is preferred that the workstation is (or comprises) a laboratory apparatus according to the first preferred embodiment, where the magnetic tool device is arranged movable with respect to the base of the apparatus. Such a workstation can comprise an internal multi-function station, which is configured to simultaneously perform multiple (more than one) work-steps on the sample(s).

The method according to the invention for performing a work step on at least one fluid sample using a magnetic field, in particular using the laboratory apparatus according to the invention or the magnetic tool device and/or the sample reception device according to the invention, preferably comprises at least the following steps:
- arranging at least one receptacle containing a fluid sample in at least one sample reception zone of a sample reception section, wherein the at least one sample reception zone is arranged along a horizontal plane side by side with at least one engagement zone of the sample reception section;
- providing the magnetic tool device, which comprises at least one engagement element;
- moving the at least one engagement element relative to the at least one engagement zone from a first position to a second position along said horizontal plane;
- engaging the at least one engagement element with the at least one engagement zone at least in the second position, such that at least one sample reception zone is facing the side surface of the at least one engagement element;
- performing the work step on the at least one fluid sample by applying a magnetic field, in the second position.

In accordance with the present invention, no restrictions exist in regard to the term "to comprise", which is not limited to the meaning of "geometrically encompassing", in particular.

Other preferred method steps can be derived from the description of the functions of the apparatus according to the invention.

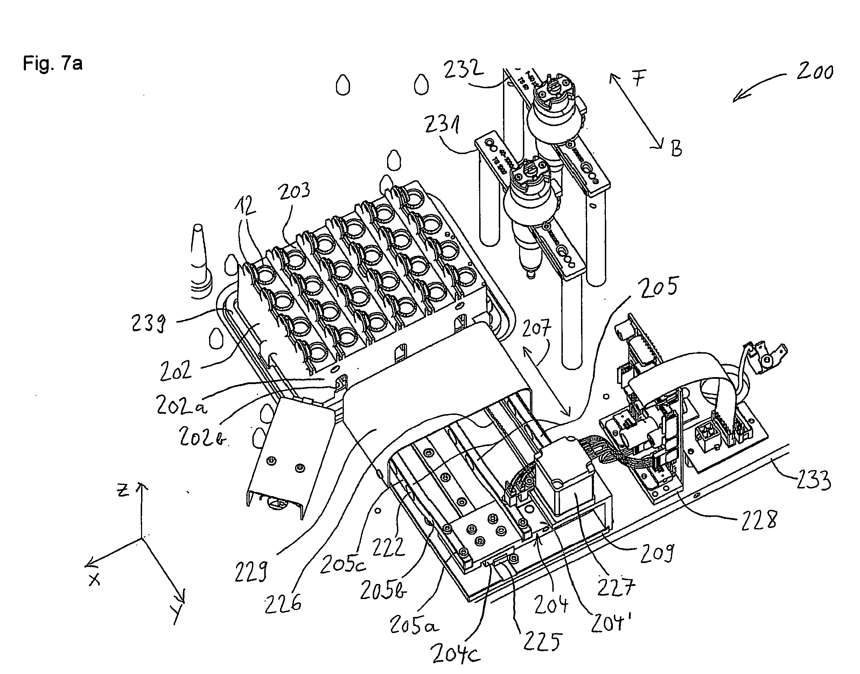

Moreover, further advantages, features and applications of the present invention can be derived from the following embodiments of the apparatus and the method according to the present invention with reference to the drawings. In the following, equal reference signs substantially describe equal devices. Regarding the figures 7a, 7b, 8, 9, 10a, 10b, 10c and 10d, preferred relative measures of the apparatus according to the invention and its components can be derived from said figures, by comparison.

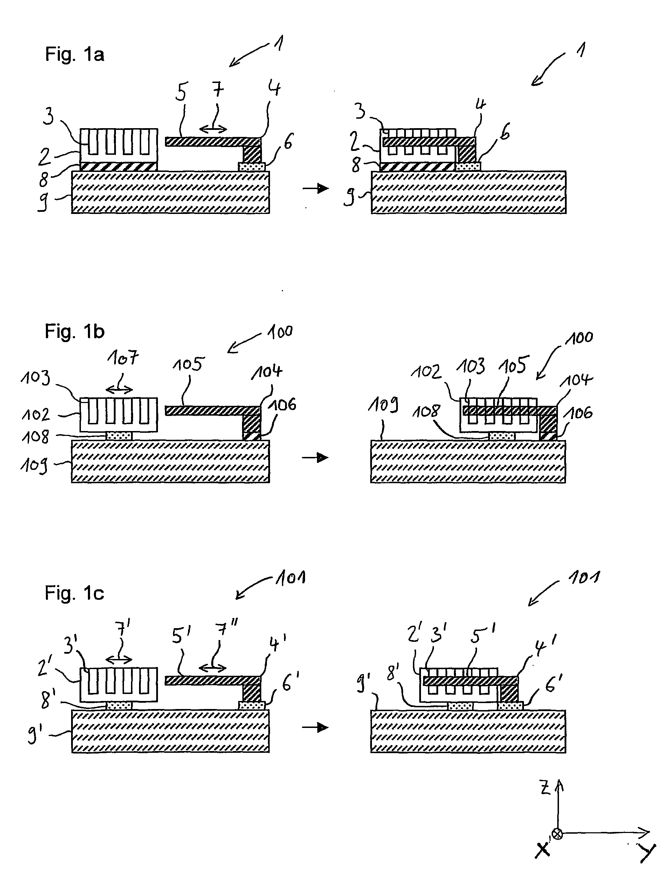

Fig. 1 a schematically shows a side view of first preferred embodiment of the laboratory apparatus according to the invention.
Fig. 1 b schematically shows a side view of a second preferred embodiment of the laboratory apparatus according to the invention.
Fig. 1 c schematically shows a side view of a third preferred embodiment of the laboratory apparatus according to the invention.
Fig. 2 schematically shows a side view of another preferred embodiment of the laboratory apparatus according to the invention.
Fig. 3a schematically shows a top view of the sample reception section and the magnetic tool device according to a preferred embodiment of the apparatus according to the invention, in the first position.
Fig. 3b shows a top view of the components of Fig. 3a, in the second position.
Fig. 3c schematically shows a top view of the sample reception section and the magnetic tool device according to a preferred embodiment of the apparatus according to the invention, in the first position.
Fig. 3d shows a top view of the components of Fig. 3c, in the second position.
Fig. 3e schematically shows a top view of the sample reception section and engagement element of the magnetic tool device according to Fig. 3a and 3b, in six different relative positions, referred to as positions I - VI of the sample reception section and the engagement element.
Fig. 3f schematically shows a top view of the sample reception section and engagement element of the magnetic tool device according to another preferred embodiment of the apparatus according to the invention, in the first position I' and the second position II'.
Fig. 4a schematically shows a top view of the sample reception section and the magnetic tool device according to a preferred embodiment of the apparatus according to the invention, in the first position.
Fig. 4b shows a top view of the components of Fig. 4a, in the second position.
Fig. 5a schematically shows a top view of the sample reception section and the magnetic tool device according to a preferred embodiment of the apparatus according to the invention, in the second position.
Fig. 5b schematically shows a side view of the sample reception section and the magnetic tool device according to another preferred embodiment of the apparatus according to the invention, in the second position.
Fig. 5c schematically shows a side view of the sample reception section and the magnetic tool device according to another preferred embodiment of the apparatus according to the invention, in the second position.
Fig. 5d shows a side view of an embodiment of a sample reception device according to the invention.
Fig. 5e shows the embodiment of a sample reception device in Fig. 5d with sample receptacles.
Fig. 5f schematically shows a side view of the sample reception section according to Fig. 5d and Fig. 5e and the magnetic tool device according to Fig. 5b.
Fig. 6 schematically shows a perspective view of the sample reception section and the magnetic tool device according to another preferred embodiment of the apparatus according to the invention, in the first position.
Fig. 7a schematically shows an isometric perspective view of the laboratory apparatus according to the first preferred embodiment of the apparatus according to the invention, in the first position.
Fig. 7b shows an isometric perspective view of the laboratory apparatus of Fig. 7a, in the second position.
Fig. 8 schematically shows an isometric perspective view of the laboratory apparatus according to the second preferred embodiment of the apparatus according to the invention, in the first position.
Fig. 9 schematically shows an isometric perspective view of the laboratory apparatus according to the first preferred embodiment of the apparatus according to the invention, which is an automated workstation.
Fig. 10a shows a side view of a detail of the laboratory apparatus of Fig. 9, in the first position, with a pipette tool in an upper position.
Fig. 10b shows a side view of a detail of the laboratory apparatus of Fig. 9, in the second position, with a pipette tool in an upper position.
Fig. 10c shows a side view of a detail of the laboratory apparatus of Fig. 9, in the second position, with a pipette tool in an upper position.
Fig. 10d shows a side view of a detail of the laboratory apparatus of Fig. 9, in the first position, with a pipette tool in an upper position.

Fig. 1 shows a configuration of a first preferred embodiment of the laboratory apparatus according to the invention, which is the laboratory apparatus 1. The laboratory apparatus has at least the function of performing a work step on a plurality of samples, by applying a magnetic field. The apparatus 1 comprises a base 9, which is a platform. The at least one sample reception section, being provided by the sample reception device 2, is stationary arranged and mounted by connection means 8 to the base 9, while the magnetic tool device 4 is movable with respect to the base 9 by using a transport device 6. The transport device can make the magnetic tool device to move along a straight horizontal direction 7, which is parallel to the same plane, along which the sample reception zones 3 are aligned. Said plane is perpendicular to the drawing surface. Each sample reception zone 3, being a hollow space in the sample reception device 2, can receive a sample receptacle (not shown). On the left side of Fig. 1a, the sample reception device 2 and the magnetic tool device 4 are in the first position. The engagement element 5 does not engage any engagement zone, e.g. a hollow space, between the sample reception zones 3. On the right side of Fig. 1 a, the sample reception device 2 and the magnetic tool device 4, including the engagement element 5, are in the second position. The engagement element 5 does engage an engagement zone, here a hollow space, between some of the sample reception zones 3.

Fig. 1 b shows a configuration of the second preferred embodiment of the laboratory apparatus 100. The sample reception section, being provided by the sample reception device 102, with the sample reception zones 103 is movable with respect to the base 109, using the transport device 108 for causing a movement along a straight horizontal direction 107. The magnetic tool device 104 is stationary with respect to the base, using the connection member 106. The magnetic tool device 104 is a station in the pathway of an automated multi-step process of treatment of samples, where the non-invasive treatment station is one of multiple stations, where a work step on the samples is performed. On the left side of Fig. 1 b, the sample reception device 102 and the magnetic tool device 104 are in the first position. The engagement element 105 does not engage any engagement zone between the sample reception zones 103. On the right side of Fig. 1a, the sample reception device 102 and the magnetic tool device 104 are in the second position. The engagement element 105 does engage an engagement zone, here a hollow space, between some of the sample reception zones 103.

Fig. 1c shows a configuration of the third preferred embodiment of the laboratory apparatus 101. The laboratory apparatus 101 comprises a base 9', wherein the sample reception section, being provided by the sample reception device 2', and the magnetic tool device 4' are both movable with respect to the base, using the transport devices 8' and 6', for causing movements along a straight horizontal direction 7' and 7", respectively. The left side of Fig. 1c shows the first position and the right side of Fig. 1c shows the second position, where the engagement element 5' engages an engagement zone, here a hollow space, between the sample reception zones 3'.

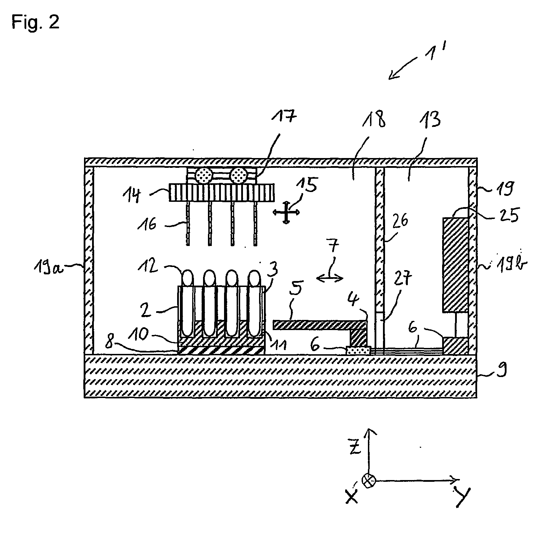

The laboratory apparatus according to the embodiments of Fig. 1 a, 1b and 1 c offer the advantage that the receptacles (sample vessels, e.g. tubes), which are located in the sample reception section, can be easily treated from the top, e.g. by a pipetting tool, while at the same time changing the position of the magnetic tool device, thereby controlling the performance of the non-invasive treatment during the additional work step of pipetting. Similarly, another work step, for example a temperature control, can be performed easily from the bottom side of the sample reception section, while at the same time changing the position of the magnetic tool device. Such a possibility is shown in Fig. 2, which is a laboratory apparatus 1' according to the first embodiment, which contains similar components as shown in Fig. 1a.

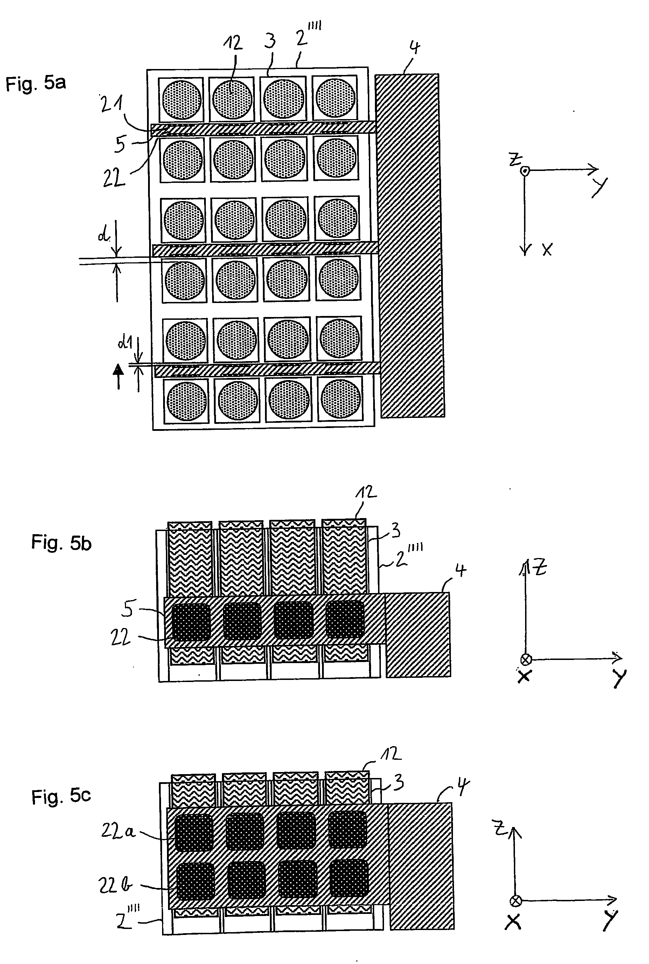

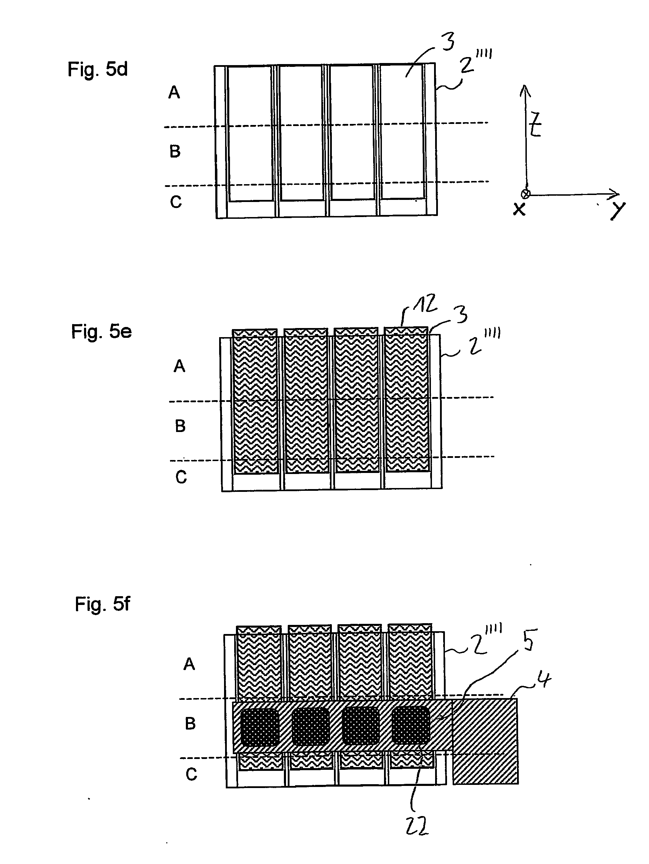

Fig. 2 shows a side view of the laboratory apparatus 1'. The sample reception section is provided by a sample reception device 2, which is further configured to be a thermorack 10, which is stationary mounted by connection means 8 to the base 9 and is used to control the temperature of the samples in the single sample tubes 12, which are held by the recesses 11 in the top surface of the thermorack 10. Arranged above the sample reception section 2 is the pipetting tool 14, which has pipette tips 16 for aspirating liquid from the samples or for distributing liquid to the samples. The pipetting tool is suspended to the frame 19 and is movable in all directions 15 by the transport device 17, which is a transport and lift device. The pipetting tool is configured for transporting liquid between the position of the sample tubes and other positions or stations (not shown) of the apparatus. Due to the fact that the relative motion 7 takes place along a horizontal plane, the engagement element 5 can engage the sample reception section 2 from the side and enter an intermediate area of the sample reception section 2. The intermediate area is stacked vertically between an upper area and a bottom area, see description of Fig. 5a, 5b, 5c, 5d, 5e and 5f. Therefore, enough space is available in the bottom area of the sample reception section to provide further devices, here a bottom part of the thermorack 10. Analogous, enough space is available in the upper area of the sample reception section 2 to operate the pipetting tool 14 and to access the sample tubes 12 from above. The transport device 6 is arranged to move the magnetic tool device 4 along the straight horizontal direction 7 from a starting position, which is a first position, to the position of engagement ("second position").

The laboratory apparatus 1' is a laboratory automated work station 1' for performing work steps, in particular a magnetic work step on the at least one sample. The laboratory apparatus 1' comprises a programmable electric control device 25, which comprises means for automatically performing work steps, in particular a magnetic work step, a work step of temperature control of the at least one sample or other work steps on the at least one sample. Said means can comprise programmable electrical circuitry and/or can comprise a computer program code for automatically performing the work step(s).

The inside of the frame 19 comprises at least two compartments. A compartment 18 is the sample treatment compartment and the compartment 13 is the control compartment. The sample treatment compartment and the compartment 13 is the control compartment, which contains the control device 25. The baffle 26 separates the compartments 13, 18 from each other. An opening 27 in the baffle 26 connects the compartments 13, 18 and allows the magnetic tool device 4 to be transferred from a starting position (a "first position"), where the magnetic tool device 4 is arranged at least partially, preferably substantially completely, within the control compartment 13, to the second position, where the magnetic tool device 4 is arranged at least partially, preferably substantially completely, within the sample operation compartment 18. Generally, separating the inside into at least two compartments offers the advantage, that the compartments are protected against each other. For example, humidity and actions occurring in the sample operation compartment can be restricted to the compartment 18, while lost heat and dust from the control compartment 13 can be restricted to said compartment 13. Moving the magnetic tool device 4 to the parking position, (here: the starting position), where the magnetic tool device 4 is arranged at least partially, preferably substantially completely, within the control compartment 13, offers the advantages that the magnetic tool device 4 is protected against environmental influences from the sample operation compartment and that the sample operation compartment 18 gains more free space for moving and operating the samples.

Moreover, the control compartment 13 is arranged in the back part of the inside of the apparatus 1'. The baffle 26 can form a rear panel of the sample operation compartment 18. The rear panel 19b, which is assigned to the frame 19, can be configured to be removable or openable for accessing the control compartment 13, e.g. for maintenance reasons. This way, a contamination of the sample operation compartment 18 can be avoided. The front side 19a is accessible for the user, for placing and removing samples and accessories for the apparatus. Having the control compartment 13 being located in the back part allows an easier access to the sample operation compartment 18.

Fig. 3a shows a top view of the sample reception section 32 and the magnetic tool device 34 of the laboratory apparatus 31 according to the invention, in the first position. The arm section of the magnetic tool device 34 comprises one arm 35. The relative movement 37 causes the at least one engagement element to engage the at least one engagement zone 20 while moving in parallel relative to the sample reception zones 33, until the second position is reached (Fig. 3b). The arm 35 has four magnet elements (not shown) mounted on one of the two opposing face sides of the arm 35. One arm 35 and one relative movement can be used to efficiently provide a magnetic work step on multiple samples (here: four). However, the invention is advantageous also if the sample reception section comprises only one sample reception zone 33. The engagement element 35 has two opposing side surfaces and one front surface 35", which is smaller than a side surface. The magnet elements are mounted such that, in the second position shown in Fig. 3b, each magnet element is facing one receptacle 12, which is located in a sample reception zone 33, when the arm has engaged the engagement zone 20 of the sample reception section 32. The face sides of the engagement element 35 have a larger surface for arranging a larger magnet element than has the front side 35", in particular. Therefore, the magnetic effect, which is applied to the sample in the receptacle 12, is more efficient.

Fig. 3c shows the laboratory apparatus 31', wherein the engagement element 35' has eight magnet elements (not shown), mounted on both side surfaces of the arm 35'. One arm 35 and one relative movement can be used to efficiently provide a magnetic work step on multiple samples (here: eight) in the second position (Fig. 3d).

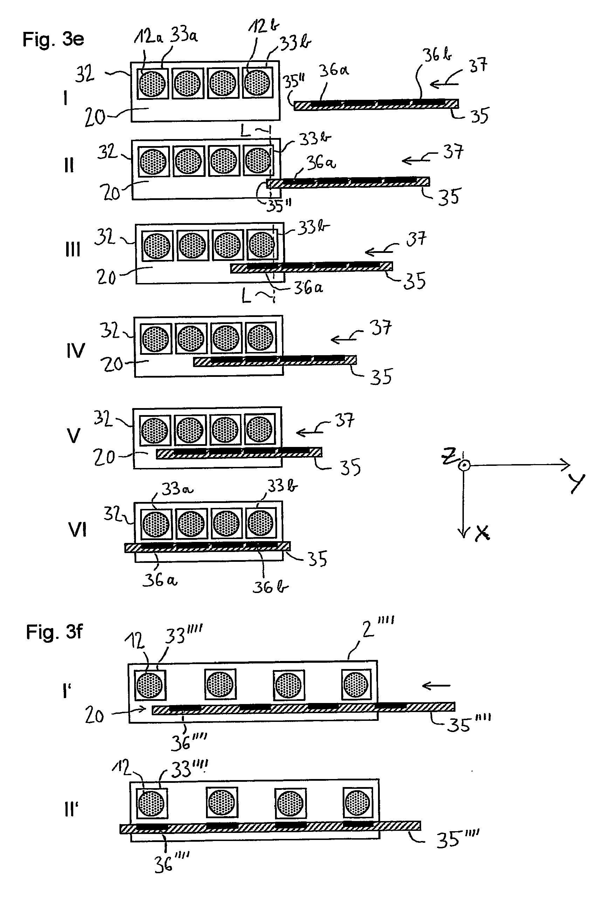

Fig. 3e schematically shows a top view of the sample reception section and engagement element of the magnetic tool device according to Fig. 3a and 3b, in six different relative positions, referred to as positions I - VI of the sample reception section and the engagement element. Positions I and II are positions, where the engagement element 35 is not arranged relative to the at least one engagement zone 20 for performing a work step using a magnetic field, which, respectively, is a "first position" according to the invention. In position I, the engagement element 35 does not engage the engagement zone 20 of the sample reception section 32, while in position II, the engagement element 35 engages the engagement zone 20 at least in part. Preferably and in general, the paths of the engagement element to the sample reception section during the relative motion are in parallel. This is the case here: the paths of the engagement element 35 to the sample reception section 32 during the relative motion 37 are in parallel.

In position III of Fig. 3e, the engagement element 35 is engaging with the engagement zone 20 at least in part and is arranged for performing a work step using a magnetic field in the second position. In particular, the magnet element 36a, e.g. a permanent magnet, is facing the sample reception zone 33b, in position III, while this is not the case in position II. In the case that the magnet element is facing the sample reception zone (e.g. in position III-VI), a magnetic work step can be performed between a sample with magnetic material, which is arranged in a receptacle located in the sample reception zone, wherein said work step can preferably not be applied in a position (e.g. position II), where the magnet element is not facing the sample reception zone, because, for example, the magnetic force may be too small for performing the magnetic work step. Therefore, position III is a "second position" according to the invention, which is also the case for positions IV, V and VI. Starting from the starting position I, which is a first position, the front surface 35" of the engagement element 35 does not move toward the sample reception zone 33b, but moves toward the engagement zone 20. In position II, namely between the first position and the second position, the engagement element 35 moves at least partly in parallel to the sample reception zone 33b during the relative motion 37. This is a general aspect of the relative motion of the at least one engagement element and the sample reception section of the apparatus according to the invention. Generally, "moving in parallel" of at least a part of the engagement element and at least a sample reception zone during a relative motion preferably means that at least one straight line L in the horizontal plane, which is perpendicular to said relative motion, crosses the sample reception zone and also crosses the engagement element.

Position VI is the target position or end position of the relative motion, where a maximum number of magnet elements of the at least one engagement element is facing a respective sample reception zone, which is assigned to the respective magnet element. In the embodiment, each magnet element of the engagement element is facing a sample reception zone. In the target position, a magnetic work step can be performed between each pair of magnet element and sample reception zone, which is assigned to a magnet element, e.g. between pairs (36a; 33a) and (36b; 33b).

Fig. 3f schematically shows a top view of the sample reception section 2"" and engagement element 35"", in the first position I' and the second position II'. In both positions, the engagement element is at least in part engaging the engagement zone 20. In position I', the magnet element 36"" is not facing the assigned sample reception zone 33"" and is not arranged for performing a magnetic work step, because the magnetic force may be too small for performing the magnetic work step. In contrast, in position II', the magnet element 36"" is facing the assigned sample reception zone 33"" and is arranged for performing a magnetic work step, because the magnetic interaction between the magnetic material in the sample 12 may be strong enough to effect the magnetic work step.

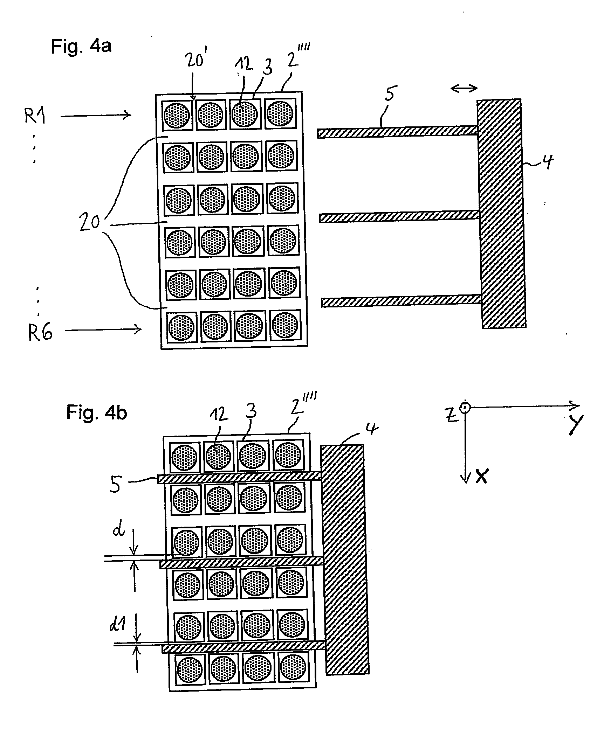

Fig. 4a shows a top view of a sample reception section 2"" and the magnetic tool device 4 according to a preferred embodiment of the apparatus according to the invention, in the first position. The sample reception section 2"" has a number N of 4 * 6 = 24 = N sample reception zones 3 for receiving the same number of sample receptacles 12. The sample reception zones are arranged in 6 rows R1 to R6, each row containing 4 sample reception zones. The 6 rows of sample reception zones are separated by five hollow spaces 20. A hollow space, in this case, has a substantially cuboid-shaped section, which is configured to receive the engagement element 5 in a fitting manner. Here three hollow spaces form an engagement zone, repectively. Further, the five hollow spaces 20 are connected by the gap spaces 20' between the adjacent sample reception zones of a row. Therefore, the five hollow spaces are forming a continuous hollow space. Therefore, the hollow space can also be formed by several hollow space sections, which are connected.

The magnetic tool device has an arm section 4, 5 with three arms 5, which form the engagement elements 5. The arrangement, where the number of arms is half of the number of rows of reception zones, is preferred, because it requires less effort for providing and (relatively) positioning the magnetic tool device.

In the second position in Fig. 4b, each engagement element 5 is positioned in a minimal distance d1 with respect to the adjacent sample reception zones 3 of a row and, respectively, in a minimal distance d to the sample receptacle 12. This way, the non-invasive interaction of the engagement elements with the fluid samples is maximized by using the short distance d (or d1). The interaction is based on a magnet field, in the present embodiments of the apparatus according to the invention.

Fig. 5a shows a top view of the sample reception section 2"" and the magnetic tool device 4 of Fig. 4b, in the second position. The sample reception section 2"" can be provided by a sample reception device. Further shown is a number of N1=N=24 permanent magnets, which are connected to the engagement elements, namely the arms 5 of the magnetic tool device 4. The permanent magnets are arranged such at the arms 5 that a number of N2 pairs 21, 22 of permanent magnets are presented, which are arranged on opposing side faces of the arms 5. Preferably, the pairs of permanent magnets are spaced apart by a distance, which corresponds to the distance of the vertical center lines of adjacent sample reception zones 3 of one row. Here, this distance is equal for all pairs of permanent magnets.

All permanent magnets shown have a poling in the same direction, as indicated by the arrow S-N, which indicates a main axis of the magnet field of the individual permanent magnets. This has the advantage, amongst others, that the magnets of a pair of magnets do attract themselves, which is another support and stabilization for the connection of the magnets at the engagement elements. However, it is also possible to arrange the permanent magnets in another pattern of differing orientations. A permanent magnet 21 or 22 has a plate-like shape, preferably substantially a cuboid shape, or a cylindrical shape, wherein the poling is oriented vertically to the main surface of the plate. Depending on the strength of the magnet, the diameter of the permanent magnet can be varied. The diameter can be smaller than the diameter of the sample reception zone, which can be 30 mm, and can be larger than 1 mm.

Fig. 5b shows how the engagement elements (arms) 5 of the magnetic tool device 4 are preferably arranged with respect to a height of the sample reception section 2"". One sample receptacle 12 is arranged, respectively, in the sample reception section 3, being a hollow space in the sample reception device 2. Preferably, the engagement elements are arranged such that they are opposing at least the lower half of a sample receptacle, e.g. sample tube 12, in the second position. Preferably, the permanent magnet are arranged such that they exert a sufficient magnetic force to magnetic material in the liquid samples, which allows to reliably separate the magnetic material from the sample liquid by concentrating the magnetic material at the inner side of the sample receptacles 12, which is facing the permanent magnet. The horizontally lateral arrangement of the sample receptacles and the magnets have the advantage, that a more 2-dimensional distribution of the magnetic material on the inner side of said wall of the receptacle can be achieved, compared to prior art arrangements with magnets positioned under the receptacles. Such a 2- dimensional distribution makes it easier to wash the magnetic material, e.g. with ethanol, and to dry them afterwards.

Preferably, the arms 5 can also be moved vertically along the arm section 4. This way, the non-invasive interaction, e.g. the magnet filed, can be directed to different positions or levels in the sample receptacle such that the interaction is more efficiently applied. In particular, the magnetic material can captured more completely from the solution. Generally, in the case that the engagement elements are configured to be movable in the direction perpendicular or angled to the plane of the relative movement, the following method steps of separating magnetic material from a fluid sample with a tool section with engagement elements and with magnet elements are provided, which is in particular useful for sample receptacles with a relatively large length: i) positioning the magnet elements at the upper border of the sample receptacles; ii) moving the magnet elements down to the bottom of the receptacle for collecting the magnetic material, which is spread along the height of the sample in the receptacle; iii) driving back up again, for cleaning the bottom of the receptacle from the magnetic material.

The configuration of Fig. 5c provides to use at least two magnet elements or permanent magnets 22a, 22b, which are arranged side-by-side, or as shown, on top of each other. Also such a configuration provides that the non-invasive interaction, e.g. the magnet filed, can be directed to different positions or levels in the sample receptacle such that the interaction is more efficiently applied. In particular, the magnetic material can captured more completely from the solution.

Fig. 5d shows a side view of an embodiment of a sample reception device according to the invention. The sample reception section is the section of the apparatus, which provides the spaces for arranging the sample receptacles 12, which spaces are referred to as sample reception zones 3. Therefore, the sample reception device, being a part of the apparatus, in particular a removable part of the apparatus, provides the sample reception section 2"". The sample reception section comprises an upper area A, an intermediate area B and a lower area or bottom area C. Said areas are cuboid-shaped sections of the sample reception section 2"", which are stacked on top of each other in vertical direction Z. As can be seen in Fig. 5f, the intermediate area B of the sample reception section can be used for providing the engagement zones, for engaging the at least one engagement element 5 with the at least one engagement zone in the intermediate area only. An advantage of such a configuration is that the bottom area C is accessible for other devices of the apparatus, e.g. a heat transfer section or a heat transfer device for controlling the heat of the at least one sample reception device and/or sample receptacle and/or sample. Moreover, the upper area is accessible for other devices, e.g. a fluid transfer device for transferring fluid sample through the upper openings of sample receptacles, being arranged in the sample reception section. This way, the available space of the apparatus is efficiently used.

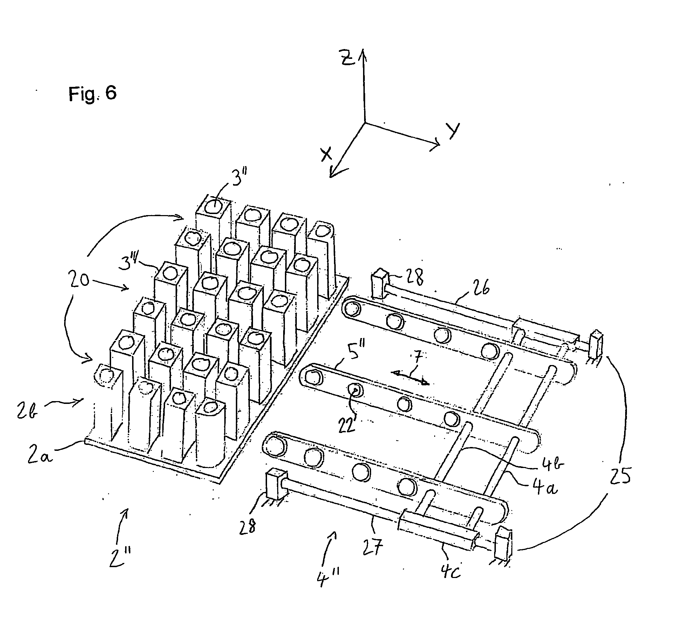

Fig. 6 shows a preferred configuration of a sample reception section 2" and a magnetic tool device 4", which are shown in the first position. The corresponding apparatus is configured according to the first preferred embodiment, shown in Fig. 1a. The sample reception section 2" is configured to be formed by a thermorack 2. The thermorack 2 has a bottom plate 2a of metal and a block section 2b, which comprises 24 sample reception zones 3". The sample reception zones 3" are provided by elongated cuboids 3"' of metal, which are oriented vertically upright along their length axis and welded to the bottom plate 2a. The main surface of the bottom plate 2a defines the plane parallel to the horizontal x-y-plane of the Cartesian coordinate system, along which the relative movement 7 of the takes place. A thermorack is useful for setting a desired temperature to biological or biochemical samples, which can provide an appropriate reaction temperature, which is required, for example, for PCR (polymerase chain reaction) or an appropriate incubation temperature for living cells. Peltier elements and temperature sensors can be attached to the base plate 2a, for example, for controlling the temperature by means of electric closed loops.

Each sample reception zone 3" is a recess in the cuboid 3"' for receiving a sample receptacle (not shown). The shapes of the recess and of the receptacle are adapted such that a possibly large fraction, between 50% and 100%, of the outer wall of the sample receptacle is contacted by the inner walls of the recess. Thereby, the thermal coupling between receptacles and the thermorack is more efficient.

The tool section 4" in Fig. 6 comprises two carrier rods 4a and 4b, which are mounted spaced apart and in parallel and form a base element, which carries the three arms 5", which are the engagement elements 5" for engaging with the three engagement zones 20, here the hollow spaces 20. Eight disc-shaped permanent magnets 21, 22 are pairwise mounted on opposing face sides of an arm 5", for facing one sample receptacle, each, in the second position. The movement of the tool section 4" with respect to the stationary sample reception section 2" is guided by the guiding device 25, which comprises the two guiding rods 26, 27, which are arranged in parallel to the direction of the relative motion 7 and which are supported on sockets 28, being stationary mounted to a base or support. The magnetic tool section 4" comprises complementary bearing means 4c with cylinder-shaped guiding bores, which receive the guiding rods, for allowing a possibly friction-reduced sliding of the tool section along the direction 7.

Fig. 7a shows a perspective view of the laboratory apparatus 200 according to the first preferred embodiment, in the first position. The laboratory apparatus corresponds to the configuration of the apparatus in Fig. 4a, 4b, 5a, 6. The apparatus can be an automated pipetting apparatus and can be part of an automated workstation. The automated apparatus or workstation has the additional function of performing a step of magnetic separation of magnetic material in sample solutions. The apparatus has a sample reception device 202, which is formed analogous to the sample reception device 2 in Fig. 2, and which is a thermorack 202, which has 5 hollow spaces 20, wherein three hollow spaces 20 are configured to be engaged by the arms 205 of the arm section 204, thereby forming an engagement zone.

The magnetic tool device 204 has, acting as a base element, a carrier plate 204', to which the three arms 205 are mounted self-supporting and extending laterally and horizontally from the side of the base element 204'. The magnetic tool device 204 is configured to form an arm section 205, here consisting of the three arms 205. Here, the arm section is configured such that the engagement elements, namely the arms 205, do not have a continuously straight shape. Rather, the engagement elements 205 have a straight mounting section 205a, which is aligned in parallel to the direction of the movement 207 of the magnetic tool device 204 and which is mounted to the base plate 204. The engagement elements 205 further have a slightly angled section 205b, which is integrally connected to the section 205a and which is aligned in an angle 0°<α<= 90° to the direction 207. The engagement elements 205 further have a straight engagement section 205c, which is integrally connected to the section 205b and which is aligned in parallel to the direction 207. The minimal distance between the mounting sections 205a of the first arm and the third arm is smaller than the minimal distance between the engagement sections 205c of the first arm and the third arm. Therefore, the arm section 205 is more compact, when compared to the arm section 4" in Fig. 6, for example. This makes it easier to deposit the mounting sections 205a and the base plate 204 of the magnetic tool device in the apparatus 200.

In total, 24 ferromagnets 222 are provided, wherein eight magnets 222 are arranged at one engagement element 205 such that one magnet faces one sample reception zone 203, in the second position. Fig. 7a shows the apparatus in a perspective view from the upper back side of the apparatus, which means that the front side F is in the background of Fig. 7a. Fig. 7a also shows the mechanism of moving the magnetic tool device 204 with respect to thermorack 202, which thermorack is stationary at a target position, which is referred to as TMX position (thermal mixing position). The magnetic tool device 204 can be used without moving the thermorack, which holds the sample receptacles. At the same time, liquid samples can be transported by one of the two pipetting tools 231, 232, which can be arranged above the thermorack 202, similar as shown in Fig. 2. The permanent magnets 222 have a diameter of 10 mm and a thickness (or "height", with respect to the poling) of 2 mm. The magnets of each arm 205 are providing magnetic interaction to the magnetic material in the samples if two adjacent rows of sample receptacles in sample reception zones 203, similar as shown in Fig. 5a.

The mounting plate 204 is mounted on the sled element 204c of a linear guiding device, and the sled element can slide along the linear rail element 225 of the linear guiding device, which is arranged in parallel to direction 207. The step motor 227 is connected to a belt transmission 226, acting as a coupling device, which can move the magnetic tool device 204 with the arms along the direction 207. The direction of movement 207 is in parallel to the main plane of the support plate 209, which is a horizontal plane. An electronic control and supply device 228 is provided, for controlling the actions of the step motor drive 227 and providing power supply. The electronic control device 228 is connected to the central electric control device of the apparatus via a CAN-data bus. The electronic control device 228, the step motor drive 227 and, in a first position (parking position), the base plate 204 with mounting sections 205a of arms 205, are arranged in the back space of the apparatus and are not visible to the user. The thermorack 202 has openings 202b on the back face side 202a, for letting the engagement elements 205 engage the corresponding engagement zones in the thermorack 202. The part of the engagement elements 205, which is exposed to the environment, is covered by a means for covering 229, in the first position.

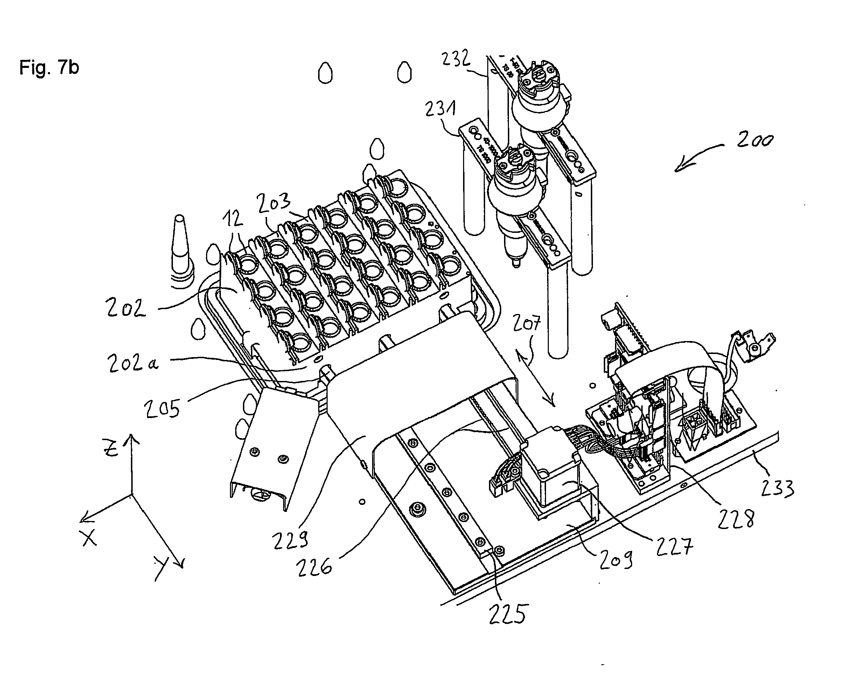

Fig. 7b shows a perspective view of the laboratory apparatus 200 of Fig. 7a, in the second position.

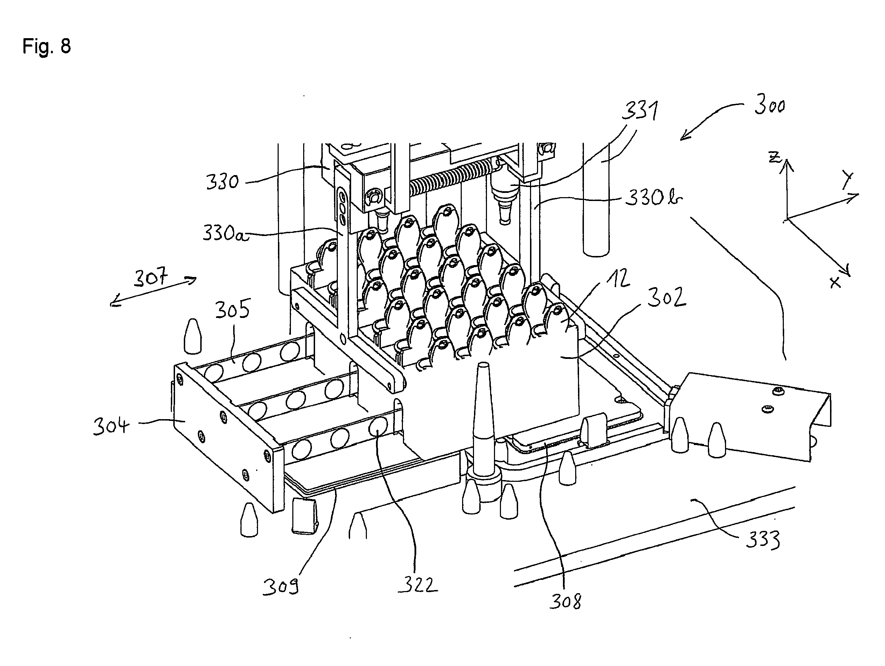

Fig. 8 schematically shows a perspective view of the laboratory apparatus 300 according to the second preferred embodiment of the apparatus according to the invention, in an intermediate position between the first position and the second position. The apparatus 300 is similar to the apparatus 200 and serves the same purposes, which is pipetting sample liquid, transporting the same and performing magnetic separation. Here, the magnetic tool device 304 with three arms 305 is unmovably mounted to the base plate 333 and therefore arranged stationary in the apparatus 300. A transport and lift device is holding a gripper tool 330 with gripper elements 330a, 330b is provided for transporting the sample reaction section or , respectively, the sample reception device 302, which is a thermorack without temperature device, between a first position, where the sample reaction device 302 is placed on the temperature plate 331 of the base of the apparatus 300, acting as a heat transfer device, and the second position, where the sample reaction device 302 is placed on the temperature plate 309 and where the arms 305 engage the engagement zones in the sample reaction section.

Below the temperature plate 308, peltier elements and temperature sensors (not shown) are attached to the respective temperature plate, for controlling the temperature by means of electric closed loops. For controlling the temperature of the thermorack 302, the thermorack 302 will be in thermal contact with the temperature plate 308 in a first position.

In any position of the thermorack 302, one of the pipetting tools 331 can be used to transport a liquid sample to one of the sample receptacles 12 (or to remove the sample from the receptacle). For this purpose, the gripper tool 330 is removed from the transport and lift device and is replaced by the pipetting tool 331.

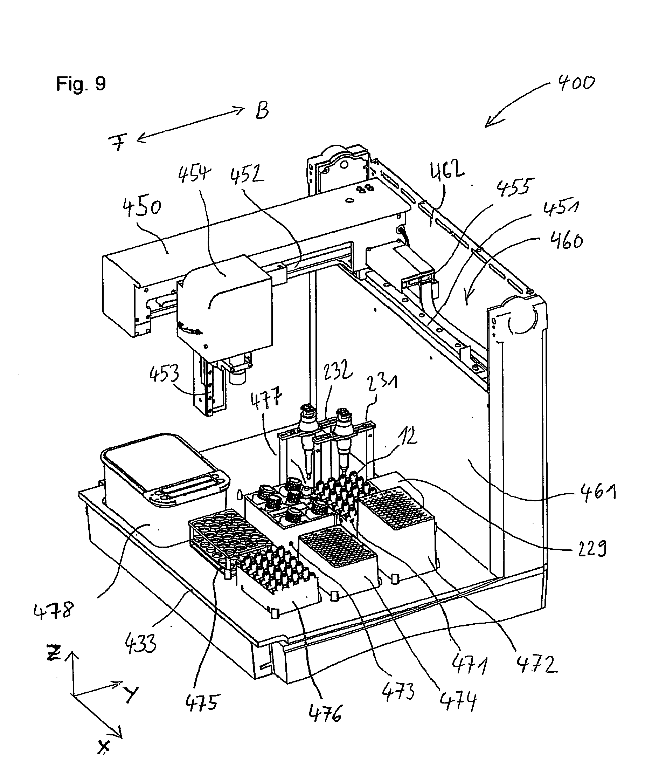

Fig. 9 schematically shows a perspective view of the laboratory apparatus 400, which is a programmable, automated workstation 400. The apparatus 400 contains all the components of the apparatus 200, shown in Fig. 7a, 7b. As an alternative (not shown), it would also be possible to incorporate the components of the apparatus 300, shown in Fig. 8, into the apparatus 400. The apparatus 400 has a base with a base plate 433 (equal to the base plate 233 in Fig. 7a, 7b) and a housing (not shown), which encases the inside of the apparatus. The inside comprises the front compartment (in the front "F") and the back compartment (in the back "B"). The front compartment is the work area of the apparatus, for working on samples. The work area contains the transport and lift device 450 and the multiple stations for performing a work step, namely the station of the TMX position 471 (in the following also referred to as station "A1"), which holds the sample reception section with the sample receptacles 12, the storage station 472 ("A2"), hosting a rack filled with 1000 ul pipetting tips, the storage station 474 ("B2"), hosting a rack filled with 50 ul pipetting tips, the station 473 ("B1") with the reagent holder, the station 475 ("C1 ") with the sample tube holder, the station 476 ("C2") with the holder for the elution receptacles and the station 477, which hosts the two pipetting tools 231 and 232 and station 478 holds a container for waste material.

In Fig. 9, the transport and lift device 450 comprises several rails 451, 452, 453, which serve to guide the motion of the tool mounting head 454 along the three axes x, y, z. The guiding member 455 runs above the closed-off inside of the back compartment 460, which is separated from the front compartment of the apparatus 400 by the rear panel 461. The rear panel 461 separates the front compartment (the work area) from the back compartment 461. The back compartment is connected to the front compartment by an opening of the rear panel, which is covered by the means for covering 229 (see also Fig. 7a, 7b; there the rear panel 461 is not shown). The opening allows the magnetic tool device 204 to be moved along the y-direction 207 through the rear panel 461. This arrangement offers the advantage that the sample area can be kept isolated from the "machine room", namely the back compartment 460, which contains the step motor 227, the electrical control and supply device 228 and other parts. The machine room can easily be serviced, if required, by removing the back wall 462 of the housing. This way, the work area of the apparatus will not be contaminated during service. Moreover, the rear panel 461 acts as an optical cover plate, which prevents the user from being distracted from working with the apparatus.

The laboratory apparatus 200, 300 and 400, in particular, have the following advantages: pipetting procedures can be easily programmed and be performed automatically, or, if desired, user defined. In addition, magnetic separation can be performed on samples containing magnetic material, e.g. magnetic beads. Typical applications, which require magnetic separation, also can require the following steps, which are typically repeated in cycles, e.g. in 3-4 cycles: i) dosing a sample liquid; ii) mixing magnetic particles with the sample liquid; iii) controlling the temperature of the sample in one or more steps; iv) performing magnetic separation. The invention, in particular apparatus 200 and 400, offers the advantage that all of said steps i) to iv) can be performed at one single position of the sample receptacles in the apparatus, which is the TMX-position. There is no need for exchanging any tool modules, because all components required are available at the TMX-position. This saves a lot of time within the application, the processes are more continuous and reproducible and the effort for programming the apparatus for running a desired application can be kept low.

Example for performing a magnetic work step on at least one fluid sample:
The following example is explained with reference to the apparatus 200, 400 of Fig. 7a, Fig. 7b, and Fig. 9, Fig. 10a, Fig. 10b, Fig. 10c and Fig. 10d.

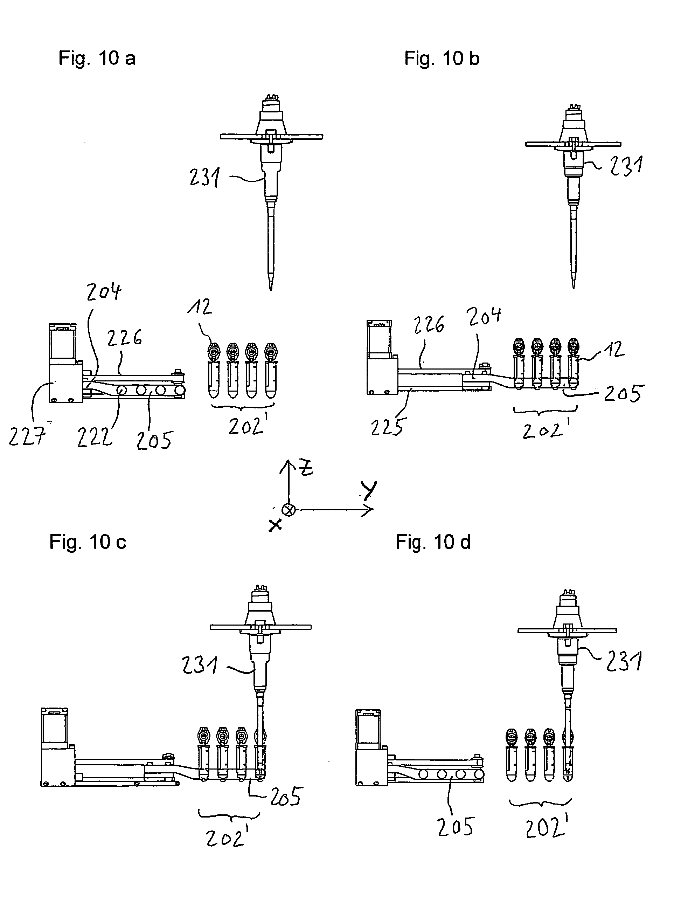

The apparatus and the method according to the invention can be used, for example, to extract RNA and DNA-fragments from blood or serum by using magnetic separation, which, first, uses the adsorption of RNA and DNA to magnetic beads, and, second, uses magnetic separation to separate the beads (with the RNA / DNA) from the solution. Reference is made to the figures 10a to 10d, which show different positions of the magnetic tool device 204 with arms 205 in relation to the sample tubes in the sample reception section 202' (not shown), which is provided by a stationary sample reception device (not shown), which is stationary held at the TMX position 471 (Fig. 9). Each figure 10a, 10b, 10c and 10d shows the same components of the apparatus 200 (Fig. 7a, 7b), and, respectively apparatus 400 (Fig. 9), in different arrangements:
Fig. 10a shows the laboratory apparatus in the first position, with the pipette tool 231 in an upper position. Fig. 10b shows the laboratory apparatus in the second position, with the pipette tool 231 in an upper position. Fig. 10c shows the laboratory apparatus in the second position, with a pipette tool 231 in an upper position. Fig. 10d shows the laboratory apparatus of Fig. 9, in the first position, with the pipette tool 231 in an upper position.

Information on performing nucleic acid-based assays, in general, can be found in US 6,605,213 B1. The following is a description of performing a nucleic acid-based assay, namely the isolation of viral DNA or RNA from cell free body fluids such as serum or plasma, by using the apparatus and the method according to the invention. Commercial kits can be used, which provide reagents and magnetic beads for isolation of samples serum or plasma, e.g. the NucleoMag® 96 Virus, -Tissue or -Blood kit from Macherey-Nagel GmbH & Co. KG, Germany.

For separating the genomic DNA (gDNA) from the blood sample, the following steps are performed:
First, the work area of the apparatus 400 has to be loaded by the user with the required accessories (reference to Fig. 9 and the description of Fig. 9): station 471 (A1, TMX position) is loaded with the rack, which contains the 24 sample receptacles 12 for performing the reactions. Station 472 (A2) and station 474 (B2) are loaded with a rack with 96 pipetting tips, respectively. Station 473 (B1) is loaded with the rack, which holds the reagent from the kit, which are required for performing the magnetic separation, e.g. NucleoMag® B-Beads, Lysis Buffer, Binding Buffer, Wash Buffer, Proteinase K, Elution Buffer. Station 475 (C1) is loaded with a rack with 24 sample tubes, each sample tube containing a different blood sample to be analyzed and treated separately. Station 476 (C2) contains the rack with 24 tubes, for receiving the elution solution.

The following steps can be performed fully automatically or semi-automatically, using any program, which is stored in the programmable electrical control device of the apparatus 400, or which can be programmed by the user, e.g. using a user interface of the apparatus 400. However, the work steps can also be at least in part user controlled.

Second,the blood sample is transferred from station C1 to A1 (one by one, using a fresh pipette tip from position A2 or B2), from one sample tube to an associated sample receptacle 12 at the position A1. For this purpose, the apparatus is operated starting in the "first position" (here: starting position of the magnetic tool device 204) in Fig. 10a and releasing (or aspirating) a liquid sample in the position in Fig. 10d.

Third, the blood cells are broken by a step of lysis. Therefore, lysis buffer is added to the sample receptacle 12 by transfer from B1 to A1. Then, proteinase K solution is added to the sample receptacle 12 by transfer from B1 to A1 and the samples in the receptacles 12 are mixed at 1200 rpm for 10 minutes, at the TMX station A1.

Fourth, the nucleic acid molecules (gDNA) from the solution are bound to the magnetic beads. Therefore binding buffer is added to the sample receptacle 12 by transfer from B1 to A1. Then, magnetic beads are added to the sample receptacle 12 by transfer from B1 to A1 and the liquid samples in the receptacles 12 are mixed at 900 rpm for 5 minutes, at the TMX station A1. The TMX station comprises means for oscillating the holding frame with the temperature plate 239 and the sample reception section 202 (thermorack with sample receptacles 12; Fig. 7a) in a substantially horizontal plane. Then, the apparatus 400 is brought to the second position (Fig. 10 b), by moving the magnetic tool device 204 towards the sample reception section 202' carrying tubes (for simplification only the tubes are shown) such that the movement 207 causes the three engagement elements 205 to engage the three engagement zones, while moving in parallel relative to the sample reception zone 203 and the sample receptacle 12. In the second position, the magnets 222 of the arm 205 are positioned such that they are facing the lowest part of the sample tube 12, respectively. This assures that the beads, which are collected at the bottom of the sample receptacle by gravity, are effectively attracted to the side face of the sample receptacle 12, which faces the magnet. Due to the construction of the apparatus according to the invention, the step of magnetic separation is highly effective. This way, the following steps of washing the inside of the sample receptacle (thereby washing the beads, which bind the nucleic acids) can be performed safely without removing the magnetic beads. The magnetic separation is performed for 2 minutes. The arms 205 are not moved in relation to the sample reception section during the magnetic separation. However, it is also possible and preferred to provide means for moving the magnetic tool device during magnetic separation. Then, the supernatant is removed from the sample receptacles 12 via the fluid transfer device (Fig. 10 c) and put to the waste container. Then, the apparatus 400 is brought back to the first position (Fig. 10 a), by moving the magnetic tool device 204 out from the sample reception section 202' designed to carry tubes (for simplification only the tubes are shown) such that the movement 207 causes the engagement elements 205 to retract from the engagement zones.

Fifth, three steps of washing the beads, which bind the nucleic acids, are performed. Washing buffer is moved via the fluid transfer device from B1 to A1 (Fig. 10d). Mixing of the beads and the washing buffer is applied at A1 at 1200 rpm for 2 minutes (Fig. 10a). The magnetic tool device is moved to the second position to hold the magnetic beads (Fig. 10 b). Magnetic separation is performed for 2 minutes (Fig. 10 b). The supernatant is removed from the sample receptacles 12 (Fig. 10 c). The magnetic tool device is removed to the first position (Fig. 10 a). Using a fresh washing buffer, the washing is repeated another two times.

Sixth, the magnetic beads with the nucleic acids are dried by waiting and/or controlled heating at A1 (Fig. 10 a).

Seventh, the nucleic acids are harvested by a step of elution. The elution provides the transfer of elution buffer from B1 to A1 (Fig. 10d), mixing the beads in the elution buffer at 1200 rpm for 10 minutes, while applying heat, thereby removing the nucleic acids from the beads; magnetically holding the beads (Fig. 10 b) for 2 minutes; removing the supernatant with the nucleic acids from the sample receptacles 12 and transferring them to the elution tubes at station C2. Purified genomic DNA from blood can directly be used for downstream applications.

## Claims

1. Laboratory apparatus (1; 1'; 31; 31'; 100; 101; 200; 300; 400) for performing a work step on at least one fluid sample in at least one sample receptacle (12) using a magnetic field, comprising
- at least one sample reception device (2; 102; 2'; 102; 202; 302), which has at least one sample reception section (2"; 2""; 32; 32'; 202'), wherein the sample reception section has at least one sample reception zone (3; 103; 3'; 3"; 33; 33'; 33""; 103; 203; 303) and at least one engagement zone (20), whereby these zones are arranged side by side along a horizontal plane, and the sample reception zone is configured to receive a sample receptacle,
- at least one magnetic tool device (4; 104; 4'; 4"; 34; 34'; 104; 204; 304), which comprises at least one engagement element (5; 105; 5'; 5"; 35; 35'; 105; 205; 305) for engaging with the at least one engagement zone (20),
- wherein the at least one sample reception section (2"; 2""; 32; 32'; 202') and the at least one engagement element are arranged movable in relation to each other between at least a first position and a second position for performing a relative movement along said horizontal plane such that during the relative movement the at least one engagement element (5; 105; 5'; 5"; 35; 35'; 105; 205; 305) and the at least one sample reception zone (3; 103; 3'; 3"; 33; 33'; 33""; 103; 203; 303) move at least partly in parallel relative to each other,
- wherein, in the first position, the at least one engagement element (5; 105; 5'; 5"; 35; 35'; 105; 205; 305) is not arranged relative to the at least one engagement zone (20) for performing a work step using a magnetic field, and
- in the second position, the at least one engagement element (5; 105; 5'; 5"; 35; 35'; 105; 205; 305) is engaging at least partly with the at least one engagement zone (20) and is arranged for performing a work step using a magnetic field in the second position.

2. Laboratory apparatus according to claim 1, wherein, in the first position, the at least one engagement element (5; 105; 5'; 5"; 35; 35'; 105; 205; 305) is not engaging the at least one engagement zone (20) and, in the second position, the at least one engagement element (5; 105; 5'; 5"; 35; 35'; 105; 205; 305) is at least partly engaging the at least one engagement zone (20).

3. Laboratory apparatus according to claim 1 or 2, which further comprises a base (9, 109, 9', 209, 309) and wherein the at least one sample reception section (2"; 32; 32'; 202') is stationary with respect to the base (9, 109, 9', 209, 309) during the relative movement, while the at least one engagement element is movable with respect to the base for performing the relative movement.

4. Laboratory apparatus according to claim 1 or 2, which further comprises a base (9, 109, 9', 209, 309) and wherein the at least one sample reception section (2"; 2""; 32; 32'; 202') is movable with respect to the base (9, 109, 9', 209, 309) for performing the relative movement, while the at least one engagement element is stationary with respect to the base during the relative movement.

5. Laboratory apparatus according to any of the previous claims, wherein the apparatus comprises holding means for holding at least one sample receptacle in the sample reception section.

6. Laboratory apparatus according to any of the previous claims, wherein the sample reception section has at least two sample reception zones, which are arranged side by side along the horizontal plane, wherein the at least one engagement zone (20) is arranged between the at least two sample reception zones.

7. Laboratory apparatus according to any of the previous claims, wherein the sample reception section has Nₐ sample reception zones, which are arranged in N_{b} parallel rows, and wherein the magnetic tool device has N_{c} engagement elements, which are configured to engage with the at least N_{d} engagement zones adjacent to the Nₐ rows in the second position, whereby N_{b}, N_{c} and N_{d} are all a natural number larger or equal than 1 and Nₐ is a natural number larger or equal than 2.

8. Laboratory apparatus according to any of the previous claims, wherein the magnetic tool device has an arm section with one or more protruding arm element, wherein each protruding arm element forms at least one engagement element.

9. Laboratory apparatus according to any of the previous claims, wherein the magnetic tool device has at least one magnet element, which is arranged at least in part at the side surface of the engagement element such that the at least one magnet element faces the at least one sample reception zone and applies a magnetic force to a magnetic material in the fluid sample in the at least one sample , receptacle, when the at least one engagement element is arranged in the second position.

10. Laboratory apparatus according to any of the previous claims, wherein the sample reception section has an upper area, an intermediate area and a bottom area, whereby the magnetic tool device is arrangeable such that, during the relative movement, the at least one engagement element engages with the intermediate area but not the upper area or the bottom area.

11. Laboratory apparatus according to any of the previous claims, which comprises a base (9, 109, 9', 209, 309) and where a heat exchange device is provided at the apparatus, which is arrangeable on the base and which comprises a heat transfer section.

12. Laboratory apparatus according to claim 10 and anyone of the previous claims, wherein the apparatus comprises a sample transport device, which comprises at least one fluid transfer device, which, in the second position, is arrangeable at least in part in the upper area.

13. Laboratory apparatus according to any of the previous claims, wherein the apparatus comprises a base (9, 109, 9', 209, 309) and wherein a sample mixing device is provided at the apparats, which is supportable on the base and is at least in part arrangeable below the sample reception section.

14. Laboratory apparatus according to any of the previous claims, wherein the apparatus has a guiding device, which is configured to guide the relative movement of the at least one sample reception section and the at least one engagement element at least between the first and second position.

15. Laboratory apparatus according to any of the previous claims, wherein the magnetic tool device is configured such that the engagement element or a part of the engagement element can also be moved in a direction perpendicular to said horizontal plane.

16. Sample reception device (2; 102; 2'; 102; 202; 302), which is arrangeable at the laboratory apparatus (1; 1'; 31; 31'; 100; 101; 200; 300; 400) according to any of the claims 1 to 15 in a target position and capable of being removed from the same and which is adapted to provide, if arranged in the target position, the sample reception section (2"; 2""; 32; 32'; 202') of the laboratory apparatus, such that the sample reception device provides at least one sample reception zone (3; 103; 3'; 3"; 33; 33'; 33""; 103; 203; 303) and at least one engagement zone (20) adapted for engagement with at least one engagement element of the laboratory apparatus, whereby these zones are arranged side by side along a horizontal plane, and the sample reception zone is configured to receive a sample receptacle.

17. Sample reception device according to claim 16, comprising a bottom plate, which is configured to be a heat transfer section, which is capable of transferring heat at least in vertical direction.

18. Magnetic tool device, in particular magnetic tool device (4; 104; 4'; 4"; 34; 34'; 104; 204; 304) of the laboratory apparatus (1; 1'; 31; 31'; 100; 101; 200; 300; 400) according to any of the claims 1 to 15, comprising at least one movable engagement element (5; 105; 5'; 5"; 35; 35'; 35""; 105; 205; 305), which is configured for performing a movement relative to a sample reception section, in particular the sample reception section (2"; 2""; 32; 32'; 202') of the laboratory apparatus (1; 1'; 31; 31'; 100; 101; 200; 300; 400) according to any of the claims 1 to 14, which sample reception section provides at least one sample reception zone (3; 103; 3'; 3"; 33; 33'; 33""; 103; 203; 303) and at least one engagement zone (20), whereby these zones are arranged side by side along a horizontal plane, and the sample reception zone is configured to receive a sample receptacle, such that during the relative movement the at least one engagement element (5; 105; 5'; 5"; 35; 35'; 35""; 105; 205; 305) and the at least one sample reception zone (3; 103; 3'; 3"; 33; 33'; 33""; 103; 203; 303) move at least partly in parallel relative to each other,
and wherein the at least one engagement element (5; 105; 5'; 5"; 35; 35'; 105; 205; 305) is configured for engaging with the at least one engagement zone (20),
wherein, in the first position, the at least one engagement element (5; 105; 5'; 5"; 35; 35'; 35""; 105; 205; 305) is not arranged relative to the at least one engagement zone (20) for performing a work step using a magnetic field, and
in the second position, the at least one engagement element (5; 105; 5'; 5"; 35; 35'; 35""; 105; 205; 305) is at least partly engaging with the at least one engagement zone (20) and is arranged for performing a work step using a magnetic field in the second position.

19. Magnetic tool device according to claim 18, which comprises an arm section, which has an arm basis and at least one arm element, which forms the at least one engagement element (5; 105; 5'; 5"; 35; 35'; 35""; 105; 205; 305) and which is protruding laterally from the arm basis in horizontal direction.

20. Magnetic tool device according to claim 19, wherein the arm section has three arm elements.

21. Magnetic tool device according to claim 18, 19 or 20, which comprises a support base, on which the at least one engagement element is supported, and which comprises a transport device for performing the relative movement of the at least one engagement element relative to the sample reception section.

22. Automated workstation, which comprises the laboratory apparatus according to claims 1 to 15, and/or the sample receptacle device according to claim 15 or 16, and/or the magnetic tool device according to claims 18 to 21, respectively.

23. Method for performing a work step on at least one fluid sample using a magnetic field, in particular using the laboratory apparatus according to claim 1 to 15 or the magnetic tool device according to claim 18 to 21, comprising at least the following steps:
• arranging at least one receptacle containing a fluid sample in at least one sample reception zone of a sample reception section, wherein the at least one sample reception zone is arranged along a horizontal plane side by side with at least one engagement zone of the sample reception section;
• providing the magnetic tool device, which comprises at least one engagement element;
• moving the at least one engagement element relative to the at least one engagement zone from a first position to a second position along said horizontal plane;
• engaging the at least one engagement element at least partly with the at least one engagement zone at least in the second position, for performing a work step using a magnetic field in the second position, in particular such that the at least one sample reception zone is facing the side surface of the at least one engagement element;
• performing the work step on the at least one fluid sample by applying a magnetic field, in the second position.
